# EUROPEAN PATENT SPECIFICATION

(11) **EP 2 916 907 B1**
(45) Date of publication and mention of the grant of the patent: **19.08.2020**
(21) Application number: 13792208.4
(22) Date of filing: 05.11.2013
(51) Int. Cl.: A61N 1/36, A61N 1/362, A61B 5/024, A61B 5/00

(54) **IMPLANTABLE NEUROSTIMULATOR FOR MANAGING TACHYARRHTHMIA AND ENHANCING HEART FAILURE PATIENT AWAKENING THROUGH VAGUS NERVE STIMULATION**
IMPLANTIERBARER NEUROSTIMULATOR ZUR BEHANDLUNG VON TACHYARRYTHMIE UND FÜR VERBESSERTES AUFWACHEN EINES HERZPATIENTEN DURCH VAGUSNERVSTIMULATION
NEUROSTIMULATEUR IMPLANTABLE POUR LA GESTION DE LA TACHYARYTHMIE ET L'AMÉLIORATION DU RÉVEIL D'UN PATIENT SOUFFRANT D'UNE INSUFFISANCE CARDIAQUE VIA LA STIMULATION DU NERF VAGUE

(30) Priority: 09.11.2012 US 201213673795; 09.11.2012 US 201213673811; 09.11.2012 US 201213673766
(43) Date of publication of application: 16.09.2015
(73) Proprietor: Cyberonics, Inc., Houston, TX 77058 (US)
(72) Inventor: LIBBUS, Imad, St. Paul, MN 55105 (US); AMURTHUR, Badri, Los Gatos, CA 95032 (US); KENKNIGHT, Bruce H., Maple Grove, MN 55311 (US)
(74) Representative: Grünecker Patent- und Rechtsanwälte PartG mbB
(86) International application number: PCT/US2013/068541
(87) International publication number: WO 2014/074523

(56) References cited:
- WO-A1-93/21824
- US-A- 5 522 854
- US-A1- 2006 100 668
- US-A1- 2012 185 007

## Description

### FIELD

This application relates in general to chronic cardiac dysfunction therapy and, in particular, to an implantable neurostimulator-implemented method for managing tachyarrhythmia, enhancing heart failure patient awakening, and enhancing post-exercise recovery through vagus nerve stimulation.

### BACKGROUND

Congestive heart failure (CHF) and other forms of chronic cardiac dysfunction (CCD) are generally attributed to an autonomic imbalance of the sympathetic and parasympathetic nervous systems that, if left untreated, can lead to cardiac arrhythmogenesis, progressively worsening cardiac function and eventual patient death. CHF is pathologically characterized by an elevated neuroexitatory state and is accompanied by physiological indications of impaired arterial and cardiopulmonary baroreflex function with reduced vagal activity.

CHF triggers compensatory activations of the sympathoadrenal (sympathetic) nervous system and the renin-angiotensin-aldosterone hormonal system, which initially help to compensate for deteriorating heart-pumping function, yet, over time, can promote progressive left ventricular dysfunction and deleterious cardiac remodeling. Patients suffering from CHF are at increased risk of tachyarrhythmias, such as atrial fibrillation (AF), ventricular tachyarrhythmias (ventricular tachycardia (VT) and ventricular fibrillation (VF)), and atrial flutter, particularly when the underlying morbidity is a form of coronary artery disease, cardiomyopathy, mitral valve prolapse, or other valvular heart disease. Sympathoadrenal activation also significantly increases the risk and severity of tachyarrhythmias due to neuronal action of the sympathetic nerve fibers in, on, or around the heart and through the release of epinephrine (adrenaline), which can exacerbate an already-elevated heart rate.

VT originates solely in the lower heart in either the ventricular tissue or Purkinje fibers. During VT, electrical signals within the ventricles begin firing abnormally and cause a rapid rate of ventricular contraction; the rate is so rapid that the heart is unable to fill properly, thereby substantially reducing the forward flow, which results in dramatic reduction in the volume of blood ejected through the aortic valve into the peripheral vascular system. This sudden reduction in blood flow can have immediate deleterious consequences since the brain and other vital organ systems require adequate blood perfusion to maintain their biological integrity. When starved of blood, even for short periods of time, vital organ systems can be damaged. The brain is particularly sensitive to reduced cardiac output. Initially, during low flow conditions, such as during VT, the brain's electrical systems are affected, and patient consciousness may be compromised. If this low or no flow condition persists for minutes, brain tissue damage begins. After six to eight minutes, this damage can become permanent and may ultimately lead to chronic impairment or death, unless the hemodynamic compromise caused by the VT is immediately and definitively corrected. During the VT, action potentials circulating in the ventricles collide and interfere with the normal propagation of action potentials from the sinoatrial (SA) node and the resulting rapid heart rate causes the heart chambers to contract prematurely and without adequately filling, thus preventing proper blood flow and causing potentially lethal hemodynamic compromise.

While other forms of tachycardia, specifically supraventricular (SVT) and sinus tachycardia, are relatively benign unless episodic or prolonged, VT can presents life-threatening risk and can degenerate into VF, asystole and sudden cardiac death. Both VT and particularly VF, as well as other forms of potentially life-threatening tachyarrhythmias, must be promptly treated to restore the heart to normal sinus rhythm. The current standard of care for treating VT includes, in order of increasing medical urgency, anti-arrhythmic medications, cardioversion, radiofrequency ablation, and heart surgery. Despite VT existing as an underlying complication of chronic cardiac dysfunction, the primary morbidity, CHF, is typically only managed through the prescription of pharmacological agents and application of dietary and lifestyle changes. As well, in contrast to the customary urgent treatment of VT, the medical measures for managing CHF are merely palliative, not curative. Patients often suffer side effects and other comorbidities, in addition to increased VT risk, due to disease progression.

The increased risk of tachyarrhythmias, particularly VT, in CHF patients is seen during the three-hour period following awakening from sleep, which is accompanied by a 2.5 times higher incidence of mortality. Tachyarrhythmias during awakening have been linked to a sympathetic activity surge that naturally occurs whenever a person is waking up as a part of the natural human circadian rhythm cycle. Sleep is characterized by the predominance of the parasympathetic system and a withdrawal of sympathetic activity, followed by a peak of sympathetic activity upon a person assuming an upright position and increasing activity level upon awakening. The surge of sympathetic activity increases heart rate, which in turn makes a CHF patient more vulnerable to the development of tachyarrhythmias that can degenerate into a life-threatening VF episode. The current standard of care for treating CCD patients relies on palliative patient management, which recognizes the risk of tachyarrhythmias occurring upon patient awakening as an unavoidable side effect of natural circadian rhythm without specific care guidelines to lessen the risk.

Heart rate naturally rises in response to exercise and other forms of physical exertion as the body's need for oxygenated blood increases. Physiologically, exercise triggers heightened sympathoadrenal activation accompanied by the release of epinephrine (adrenaline) and norepinephrine (noradrenaline), which induce sinus tachycardia and ensuing heart rate increase. In a healthy person, this physiologic response to physical exertion is countered during postexercise recovery period by parasympathetic outflow. A patient suffering from CCD, however, is at increased risk of tachyarrhythmias during the post-exercise recovery period, due to the exercise-induced exacerbation of already-increased sympathoadrenal drive. The current standard of care for treating CCD patients relies on palliative patient management, in which patients are cautioned to control the amount and degree of exercise undertaken to avoid triggering exercise-induced tachyarrhythmias and their potential sequela.

The standard of care for managing CCD in general continues to evolve. For instance, new therapeutic approaches that employ electrical stimulation of neural structures that directly address the underlying cardiac autonomic nervous system imbalance and dysregulation have been proposed. In one form, controlled stimulation of the cervical vagus nerve beneficially modulates cardiovascular regulatory function. Currently, vagus nerve stimulation (VNS) is only approved for the clinical treatment of drug-refractory epilepsy and depression, although VNS has been proposed as a therapeutic treatment of CHF in general and has been demonstrated in canine studies as efficacious in simulated treatment of AF and heart failure, such as described in Zhang et al., "Therapeutic Effects of Selective Atrioventricular Node Vagal Stimulation in Atrial Fibrillation and Heart Failure," J. Cardiovasc. Electrophysiol., Vol. pp. 1-6 (July 9, 2012).

Conventional general therapeutic alteration of cardiac vagal efferent activation through electrical stimulation targets only the efferent nerves of the parasympathetic nervous system, such as described in Sabbah et al., "Vagus Nerve Stimulation in Experimental Heart Failure," Heart Fail. Rev., 16:171-178 (2011). The Sabbah paper discusses canine studies using a vagus nerve stimulation system, manufactured by BioControl Medical Ltd., Yehud, Israel, which includes an electrical pulse generator, right ventricular endocardial sensing lead, and right vagus nerve cuff stimulation lead. The sensing lead enables stimulation of the right vagus nerve in a highly specific manner, which involves closed-loop synchronization of the vagus nerve stimulation pulse to the cardiac cycle. An asymmetric tri-polar nerve cuff electrode is implanted on the right vagus nerve at the mid-cervical position. The electrode provides cathodic induction of action potentials while simultaneously applying asymmetric anodal blocks that lead to preferential activation of vagal efferent fibers. Electrical stimulation of the right cervical vagus nerve is delivered only when heart rate increases beyond a preset threshold. Stimulation is provided at an impulse rate and intensity intended to reduce basal heart rate by ten percent by preferential stimulation of efferent vagus nerve fibers leading to the heart while blocking afferent neural impulses to the brain. Although effective in partially restoring baroreflex sensitivity and, in the canine model, increasing left ventricular ejection fraction and decreasing left ventricular end diastolic and end systolic volumes, the degree of therapeutic effect on parasympathetic activation occurs through incidental recruitment of afferent parasympathetic nerve fibers in the vagus, as well as through recruitment of efferent fibers. Efferent stimulation alone is less effective at restoring autonomic balance than bi-directional stimulation.

Other uses of electrical nerve stimulation for therapeutic treatment of various cardiac and physiological conditions are described. For instance, U.S. Patent No. 6,600,954, issued July 29, 2003 to Cohen et al. discloses a method and apparatus for selective control of nerve fiber activations. An electrode device is applied to a nerve bundle capable of generating, upon activation, unidirectional action potentials that propagate through both small diameter and large diameter sensory fibers in the nerve bundle, and away from the central nervous system. The device is particularly useful for reducing pain sensations in the legs and arms.

U.S. Patent No. 6,684,105, issued January 27, 2004 to Cohen et al. discloses an apparatus for treatment of disorders by unidirectional nerve stimulation. An apparatus for treating a specific condition includes a set of one or more electrode devices that are applied to selected sites of the central or peripheral nervous system of the patient. For some applications, a signal is applied to a nerve, such as the vagus nerve, to stimulate efferent fibers and treat motility disorders, or to a portion of the vagus nerve innervating the stomach to produce a sensation of satiety or hunger. For other applications, a signal is applied to the vagus nerve to modulate electrical activity in the brain and rouse a comatose patient, or to treat epilepsy and involuntary movement disorders.

U.S. Patent No. 7,123,961, issued October 17, 2006 to Kroll et al. discloses stimulation of autonomic nerves. An autonomic nerve is stimulated to affect cardiac function using a stimulation device in electrical communication with the heart by way of three leads suitable for delivering multi-chamber stimulation and shock therapy. For arrhythmia detection, the device utilizes atrial and ventricular sensing circuits to sense cardiac signals to determine whether a rhythm is physiologic or pathologic. The timing intervals between sensed events are classified by comparing them to a predefined rate zone limit and other characteristics to determine the type of remedial therapy needed, which includes bradycardia pacing, anti-tachycardia pacing, cardioversion shocks (synchronized with an R-wave), or defibrillation shocks (delivered asynchronously).

U.S. Patent No. 7,225,017, issued May 29, 2007 to Shelchuk discloses terminating ventricular tachycardia in connection with any stimulation device that is configured or configurable to stimulate nerves, or stimulate and shock a patient's heart. Parasympathetic stimulation is used to augment anti-tachycardia pacing, cardioversion, or defibrillation therapy. To sense atrial or ventricular cardiac signals and provide chamber pacing therapy, particularly on the left side of the patient's heart, the stimulation device is coupled to a lead designed for placement in the coronary sinus or its tributary veins. Cardioversion stimulation is delivered to a parasympathetic pathway upon detecting a ventricular tachycardia. A stimulation pulse is delivered via the lead to one or more electrodes positioned proximate to the parasympathetic pathway according to stimulation pulse parameters based at least in part on the probability of reinitiation of an arrhythmia.

U.S. Patent No. 7,277,761, issued October 2, 2007 to Shelchuk discloses vagal stimulation for improving cardiac function in heart failure or CHF patients. An autonomic nerve is stimulated to affect cardiac function using a stimulation device in electrical communication with the heart by way of three leads suitable for delivering multi-chamber endocardial stimulation and shock therapy. Where the stimulation device is intended to operate as an implantable cardioverter-defibrillator (ICD), the device detects the occurrence of an arrhythmia, and automatically applies an appropriate therapy to the heart aimed at terminating the detected arrhythmia. Defibrillation shocks are generally of moderate to high energy level, delivered asynchronously, and pertaining exclusively to the treatment of fibrillation.

U.S. Patent No. 7,295,881, issued November 13, 2007 to Cohen et al. discloses nerve branch-specific action potential activation, inhibition and monitoring. Two preferably unidirectional electrode configurations flank a nerve junction from which a preselected nerve branch issues, proximally and distally to the junction, with respect to the brain. Selective nerve branch stimulation can be used in conjunction with nerve-branch specific stimulation to achieve selective stimulation of a specific range of fiber diameters, substantially restricted to a preselected nerve branch, including heart rate control, where activating only the vagal B nerve fibers in the heart, and not vagal A nerve fibers that innervate other muscles, can be desirous.

U.S. Patent No. 7,778,703, issued August 17, 2010 to Gross et al. discloses selective nerve fiber stimulation for treating heart conditions. An electrode device is adapted to be coupled to a vagus nerve of a subject and a control unit drives the electrode device by applying stimulating and inhibiting currents to the vagus nerve, which are capable of respectively inducing action potentials in a therapeutic direction in a first set and a second set of nerve fibers in the vagus nerve and inhibiting action potentials in the therapeutic direction in the second set of nerve fibers only. The nerve fibers in the second set have larger diameters than the nerve fibers in the first set. Typically, the system is configured to treat heart failure or heart arrhythmia, such as atrial fibrillation or tachycardia by slowing or stabilizing the heart rate, or reducing cardiac contractility.

U.S. Patent No. 7,813,805, issued October 12, 2010 to Farazi and U.S. Patent No. 7,869,869, issued January 11, 2011 to Farazi both disclose subcardiac threshold vagus nerve stimulation. A vagus nerve stimulator is configured to generate electrical pulses below a cardiac threshold, which are transmitted to a vagus nerve, so as to inhibit or reduce injury resulting from ischemia. For arrhythmia detection, a heart stimulator utilizes atrial and ventricular sensing circuits to sense cardiac signals to determine whether a rhythm is physiologic or pathologic. In low-energy cardioversion, an ICD device typically delivers a cardioversion stimulus synchronously with a QRS complex; thus, avoiding the vulnerable period of the T-wave and avoiding an increased risk of initiation of VF. In general, if anti-tachycardia pacing or cardioversion fails to terminate a tachycardia, then, for example, after a programmed time interval or if the tachycardia accelerates, the ICD device initiates defibrillation therapy.

Finally, U.S. Patent No. 7,885,709, issued February 8, 2011 to Ben-David discloses nerve stimulation for treating disorders. A control unit drives an electrode device to stimulate the vagus nerve, so as to modify heart rate variability, or to reduce heart rate, by suppressing the adrenergic (sympathetic) system. Typically, the system is configured to treat heart failure or heart arrhythmia, such as atrial fibrillation or tachycardia. In one embodiment, a control unit is configured to drive an electrode device to stimulate the vagus nerve, so as to modify heart rate variability to treat a condition of the subject. Therapeutic effects of reduction in heart rate variability include the narrowing of the heart rate range, thereby eliminating very slow heart rates and very fast heart rates. For this therapeutic application, the control unit is typically configured to reduce low-frequency heart rate variability, and to adjust the level of stimulation applied based on the circadian and activity cycles of the subject. Therapeutic effects also include maximizing the mechanical efficiency of the heart by maintaining relatively constant ventricular filling times and pressures. For example, this therapeutic effect may be beneficial for subjects suffering from atrial fibrillation, in which fluctuations in heart filling times and pressure reduce cardiac efficiency.

Although delivering vagus nerve stimulation in order to prevent arrhythmias together with delivery of stimulation during awakening of a patient is known from US2012/0185007, a need remains for an approach therapeutically treating chronic cardiac dysfunction, including CHF, and cardiac arrhythmogenesis, specifically tachycardia, through a form of VNS to improve autonomic balance and cardiovascular regulatory function, to ameliorate tachyarrhythmic risk in a heart failure patient during awakening, and to enhance recovery following exercise in a heart failure patient including attenuating heart rate increase and decreasing risk of tachyarrhythmias.

### SUMMARY

While the invention is defined in the appended claims, additional embodiments that are not part of the claimed invention are discussed in the following disclosure. According to an embodiment of the present disclosure, excessive sustained activation of the sympathetic nervous system has a deleterious effect on long term cardiac performance and increases the risk of tachyarrhythmias, particularly in a patient with CCD. Bi-directional afferent and efferent neural stimulation through the vagus nerve can beneficially restore autonomic balance and improve long term clinical outcome. The neural stimulation is provided in a low level maintenance dose independent of cardiac cycle. VNS delivery can be provided through an implantable vagus neurostimulator and electrode lead, which begins delivering a restorative dose at a higher level of intensity than the maintenance dose upon sensing a condition indicative of spontaneous tachyarrhythmia. VNS maintenance dose delivery is only continually resumed if, during post-suspension monitoring, tachyarrhythmia is not found to recur.

One embodiment provides an implantable neurostimulator-implemented method for managing tachyarrhythmias through vagus nerve stimulation. An implantable neurostimulator, including a pulse generator, is configured to deliver electrical therapeutic stimulation in a manner that results in creation and propagation (in both afferent and efferent directions) of action potentials within neuronal fibers comprising the cervical vagus nerve of a patient. Operating modes are stored in the pulse generator. A maintenance dose of the electrical therapeutic stimulation is parametrically defined and tuned to restore cardiac autonomic balance through continuously-cycling, intermittent and periodic electrical pulses. A restorative dose of the electrical therapeutic stimulation is parametrically defined and tuned to prevent initiation of or disrupt tachyarrhythmia through periodic electrical pulses delivered at higher intensity, which could be higher output current, higher duty cycle, higher frequency, or longer pulse width, than the maintenance dose. The maintenance dose is therapeutically delivered to the vagus nerve independent of cardiac cycle via a pulse generator included in the implantable neurostimulator through at least a pair of helical electrodes electrically coupled to the pulse generator via a nerve stimulation therapy lead. The patient's normative physiology is monitored via a physiological sensor included in the implantable neurostimulator, and upon sensing a condition indicative of tachyarrhythmia, is switched to delivering the restorative dose to the vagus nerve via the pulse generator through the pair of helical electrodes.

A further embodiment provides an implantable neurostimulator-implemented method for managing tachyarrhythmias through vagus nerve stimulation. A maintenance dose of electrical therapeutic stimulation for delivery via an implantable neurostimulator is defined and tuned to restore cardiac autonomic balance through continuously-cycling, intermittent and periodic electrical pulses. A restorative dose of electrical therapeutic stimulation for delivery via the implantable neurostimulator is defined and tuned to prevent initiation of or disrupt tachyarrhythmia through periodic electrical pulses delivered at higher intensity, which could be higher output current, higher duty cycle, higher frequency, or longer pulse width, than the maintenance dose. Vagus nerve stimulation therapy are provided to the vagus nerve through at least a pair of helical electrodes via a stimulation therapy lead electrically coupled to a pulse generator included in the implantable neurostimulator. The maintenance dose is therapeutically delivered via the pulse generator to the vagus nerve independent of cardiac cycle in a manner that results in creation and propagation (in both afferent and efferent directions) of action potentials within neuronal fibers comprising a cervical vagus nerve of a patient. The patient's physiology is periodically monitored via a physiological sensor included in the implantable neurostimulator. Upon sensing a condition indicative of tachyarrhythmia, therapy delivery is switched to delivering the restorative dose to the vagus nerve. Upon sensing a condition indicative of a recurrence of the tachyarrhythmia, the therapeutic delivery of the restorative dose to the vagus nerve is continued via the pulse generator.

According to another embodiment of the present disclosure, excessive sustained activation of the sympathetic nervous system has a deleterious effect on long-term cardiac performance and increases the risk of tachyarrhythmias during awakening. In general, bidirectional afferent and efferent neural stimulation through the vagus nerve can beneficially restore autonomic balance and improve long term clinical outcome. Upon sensing a patient's awakening, VNS can be delivered therapeutically through an implantable vagus neurostimulator and electrode lead to a patient in an enhanced dose for a fixed period of time, absent arrhythmogenesis. The delivery of the enhanced dose following the patient's awakening helps decrease the risk of having a sudden cardiac death due to a diurinal peak in VT. Upon the expiration of the fixed period, other VNS doses can be engaged, such as a maintenance dose, if no tachyarrhythmia is present, and a restorative dose, which is delivered when the patient experiences a tachyarrhythmic event.

One embodiment provides an implantable neurostimulator-implemented method for managing tachyarrhythmias through vagus nerve stimulation. An implantable neurostimulator, including a pulse generator, is configured to deliver electrical therapeutic stimulation in a manner that results in creation and propagation (in both afferent and efferent directions) of action potentials within neuronal fibers comprising the cervical vagus nerve of a patient. Operating modes are stored in the pulse generator. An enhanced dose of the electrical therapeutic current is parametrically defined and tuned to prevent initiation of or disrupt tachyarrhythmia upon the patient's awakening from a sleep state through at least one of continuously-cycling, intermittent and periodic electrical pulses. A maintenance dose of the electrical therapeutic stimulation is parametrically defined and tuned to restore cardiac autonomic balance through continuously-cycling, intermittent and periodic electrical pulses to be delivered at a lower intensity, which could be a lower output current, lower duty cycle, lower frequency, or shorter pulse width, than the enhanced dose. A restorative dose of the electrical therapeutic stimulation is parametrically defined and tuned to prevent initiation of or disrupt tachyarrhythmia through periodic electrical pulses delivered at higher intensity, which could be higher output current, higher duty cycle, higher frequency, or longer pulse width, than the maintenance dose. The patient's physiological state is monitored using at least one sensor, such as an accelerometer to sense the patient's posture and movements, a minute ventilation sensor to monitor the patient's respiration, and a heart rate sensor to monitor the patient's heart rate. The patient's normative physiology is monitored via a physiological sensor included in the implantable neurostimulator, and upon sensing a condition indicative of tachyarrhythmia, the intensity of the enhanced dose can be increased. The increase can be progressive based on the patient's heart rate trajectory, with the intensity increasing multiple times as the tachyarrhythmia fails to respond to the VNS stimulation or the delivery of the enhanced dose can be maximized based on the patient's heart rate trajectory. If the physiological sensors detect a condition indicative of bradyarrhythmia, the enhanced dose is suspended. Upon an expiration of the period of time for delivery of the enhanced dose, the maintenance dose can be delivered if the patient is not experiencing a tachyarrhythmic episode at the moment. If the patient is experiencing tachyarrhythmia at the time the period for delivery ends, the restorative dose can be delivered.

According to another embodiment of the present disclosure, prolonged activation of the sympathetic nervous system during the post-exercise recovery period increases the risk of tachyarrhythmias, particularly in a patient with CCD. In general, bidirectional afferent and efferent neural stimulation through the vagus nerve can beneficially restore autonomic balance and improve long term clinical outcome. During non-exertion periods, VNS can be delivered therapeutically through an implantable vagus neurostimulator and electrode lead to a patient in a maintenance dose, which helps to restore the patient's cardiac autonomic balance. During exercise, VNS can be suspended. Thereafter, during the post-exercise recovery period, VNS can be delivered in an enhanced dose, which is set to a higher level of intensity than the maintenance dose to facilitate exercise recovery and lower tachyarrhythmic risk.

One embodiment provides an implantable neurostimulator-implemented method for enhancing post-exercise recovery through vagus nerve stimulation. An implantable neurostimulator, including a pulse generator configured to deliver electrical therapeutic stimulation in a manner that results in creation and propagation (in both afferent and efferent directions) of action potentials within neuronal fibers including a patient's cervical vagus nerve. An operating mode is stored in the pulse generator. An enhanced dose of the electrical therapeutic stimulation is parametrically defined and tuned to prevent or disrupt tachyarrhythmia through continuously-cycling, intermittent and periodic electrical pulses. The patient's physiological state is monitored during physical exercise via at least one sensor included in the implantable neurostimulator, and upon sensing a condition indicative of cessation of the physical exercise, the enhanced dose is delivered for a period of time to the vagus nerve.

By improving autonomic balance and cardiovascular regulatory function, therapeutic VNS operates acutely to decrease heart rate, reflexively increase heart rate variability and coronary flow, reduce cardiac workload through vasodilation, and improve left ventricular relaxation without aggravating comorbid tachyarrhythmia or other cardiac arrhythmic conditions. Over the long term, low dosage VNS provides the chronic benefits of decreased negative cytokine production, increased baroreflex sensitivity, increased respiratory gas exchange efficiency, favorable gene expression, renin-angiotensin-aldosterone system down-regulation, and anti-arrhythmic, anti-apoptotic, and ectopy-reducing anti-inflammatory effects.

Still other embodiments of the present invention will become readily apparent to those skilled in the art from the following detailed description, wherein are described embodiments by way of illustrating the best mode contemplated for carrying out the invention. As will be realized, the invention is capable of other and different embodiments and its several details are capable of modifications in various obvious respects, all without departing from the spirit and the scope of the present invention. Accordingly, the drawings and detailed description are to be regarded as illustrative in nature and not as restrictive.

### BRIEF DESCRIPTION OF THE DRAWINGS

FIGURE 1 is a front anatomical diagram showing, by way of example, placement of an implantable vagus stimulation device in a male patient, in accordance with one embodiment.
FIGURES 2A and 2B are diagrams respectively showing the implantable neurostimulator and the stimulation therapy lead of FIGURE 1.
FIGURE 3 is a graph showing, by way of example, the relationship between the targeted therapeutic efficacy and the extent of potential side effects resulting from use of the implantable neurostimulator of FIGURE 1.
FIGURE 4 is a graph showing, by way of example, the optimal duty cycle range based on the intersection depicted in FIGURE 3.
FIGURE 5 is a timing diagram showing, by way of example, a stimulation cycle and an inhibition cycle of VNS as provided by implantable neurostimulator of FIGURE 1.
FIGURE 6 is a flow diagram showing an implantable neurostimulator-implemented method for managing tachyarrhythmias through vagus nerve stimulation, in accordance with one embodiment.
FIGURE 7 is a flow diagram showing a routine for storing operating modes for use with the method of FIGURE 6.
FIGURE 8 is a flow diagram showing an optional routine for determining an onset or presence of arrhythmia with a recency filter for use with the method of FIGURE 6.
FIGURE 9 is a flow diagram showing an implantable neurostimulator-implemented method for managing tachyarrhythmias upon awakening through vagus nerve stimulation, in accordance with one embodiment.
FIGURE 10 is a flow diagram showing a routine for delivering an enhanced dose of VNS stimulation upon the patient's awakening for use with the method of FIGURE 9, in accordance with one embodiment.
FIGURE 11 is a flow diagram showing a routine for storing operating modes for use with the method of FIGURE 9.
FIGURE 12 is a flow diagram showing an implantable neurostimulator-implemented method for enhancing post-exercise recovery through vagus nerve stimulation, in accordance with one embodiment.
FIGURE 13 is a flow diagram showing a routine for storing operating modes for use with the method of FIGURE 12.

### DETAILED DESCRIPTION

Changes in autonomic control of the cardiovascular systems of patients suffering from CHF and other cardiovascular diseases push the autonomic nervous system out of balance and favor increased sympathetic and decreased parasympathetic central outflow. The imbalance is accompanied by pronounced elevation of basal heart rate arising from chronic sympathetic hyperactivation along the neurocardiac axis. Such is exacerbated during awakening by the surge in sympathetic activity that is a part of natural human circadian rhythms. Such is also exacerbated by exercise and other forms of physical exertion and follow-on recovery. Such may also arise from drawn out post-exercise recovery accompanied by prolonged heart rate elevation.

Peripheral neurostimulation therapies that target the imbalance of the autonomic nervous system have been shown to improve clinical outcomes in patients treated for three to twelve months. Specifically, bi-directional autonomic regulation therapy results in simultaneous creation and propagation of efferent and afferent action potentials within afferent and efferent nerve fibers comprising the vagus nerve. The therapy directly restores autonomic balance by engaging both medullary and cardiovascular reflex control components of the autonomic nervous system. Upon stimulation of the cervical vagus nerve, action potentials propagate away from the stimulation site in two directions, efferently toward the heart and afferently toward the brain. Efferent action potentials influence the intrinsic cardiac nervous system and the heart, while afferent action potentials influence central elements of the nervous system. Such can dampen heightened sympathetic overdrive during the post-exercise recovery period.

An implantable vagus nerve stimulator with integrated heart rate sensor, such as used to treat drug-refractory epilepsy and depression, can be adapted for use in managing chronic cardiac dysfunction through therapeutic bi-directional vagal stimulation. Such may be during patient awakening. In addition, an integrated heart rate sensor can provide continual leadless heart rate monitoring that can be used in detecting arrhythmia, particularly VT, and confirming therapeutic efficacy. An implantable vagus nerve stimulator can also be adapted for use in managing exercise-induced tachyarrhythmias in patients with CCD through therapeutic bidirectional vagal stimulation, in which the heart rate sensor provides continual heart rate monitoring that can be used in detecting cessation of physical exercise or other physical exertion to decrease risk of tachyarrhythmia, particularly VT, and confirming therapeutic efficacy.

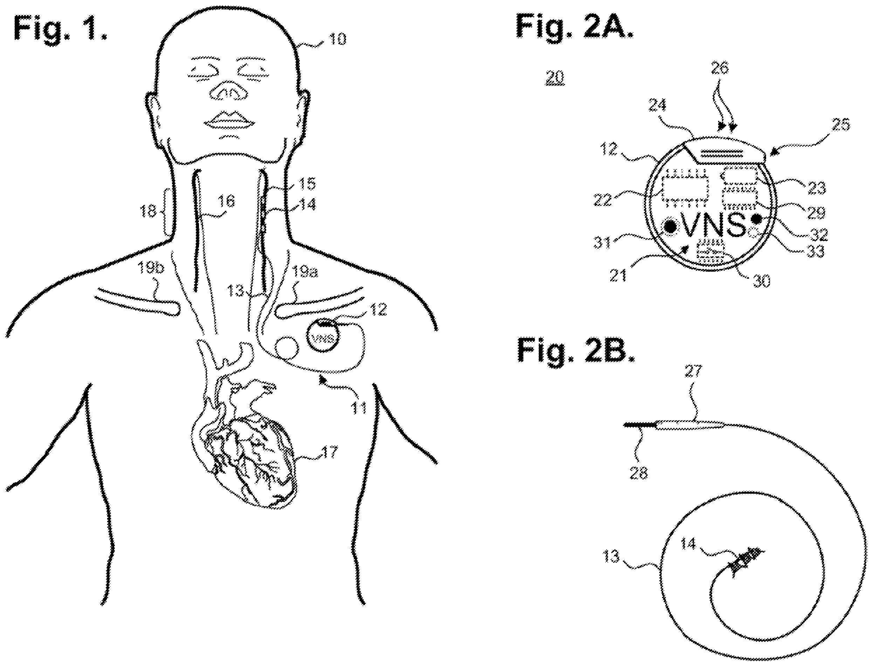

FIGURE 1 is a front anatomical diagram showing, by way of example, placement of an implantable vagus nerve stimulation (VNS) device 11 in a male patient 10, in accordance with one embodiment. The VNS provided through the stimulation device 11 operates under several mechanisms of action. These mechanisms include increasing parasympathetic outflow and inhibiting sympathetic effects by blocking norepinephrine release. More importantly, VNS triggers the release of acetylcholine (ACh) into the synaptic cleft, which has beneficial anti-arrhythmic, anti-apoptotic, and ectopy-reducing anti-inflammatory effects.

The implantable vagus stimulation device 11 includes at least three implanted components, an implantable neurostimulator 12, a therapy lead 13, and helical electrodes 14. The implantable vagus stimulation device 11 can be remotely accessed following implant through an external programmer by which the neurostimulator 12 can be remotely checked and programmed by healthcare professionals; an external magnet, such as described in commonly-assigned U.S. Patent application, entitled "Implantable Device For Facilitating Control Of Electrical Stimulation Of Cervical Vagus Nerves For Treatment Of Chronic Cardiac Dysfunction," Serial No. 13/314,130, filed on December 7, 2011, pending, for basic patient control; and an electromagnetic controller, such as described in commonly-assigned U.S. Patent application, entitled "Vagus Nerve Neurostimulator With Multiple Patient-Selectable Modes For Treating Chronic Cardiac Dysfunction," Serial No. 13/352,244, filed on January 17, 2012, pending, that enables the patient 10 to exercise increased control over therapy delivery and suspension. Together, the implantable vagus stimulation device 11 and one or more of the external components form a VNS therapeutic delivery system.

The neurostimulator 12 is implanted in the patient's right or left pectoral region generally on the same side (ipsilateral) as the vagus nerve 15, 16 to be stimulated, although other neurostimulator-vagus nerve configurations, including contra-lateral and bi-lateral are possible. The helical electrodes 14 are generally implanted on the vagus nerve 15, 16 about halfway between the clavicle 19a-b and the mastoid process. The therapy lead 13 and helical electrodes 14 are implanted by first exposing the carotid sheath and chosen vagus nerve 15, 16 through a latero-cervical incision on the ipsilateral side of the patient's neck 18. The helical electrodes 14 are then placed onto the exposed nerve sheath and tethered. A subcutaneous tunnel is formed between the respective implantation sites of the neurostimulator 12 and helical electrodes 14, through which the therapy lead 13 is guided to the neurostimulator 12 and securely connected.

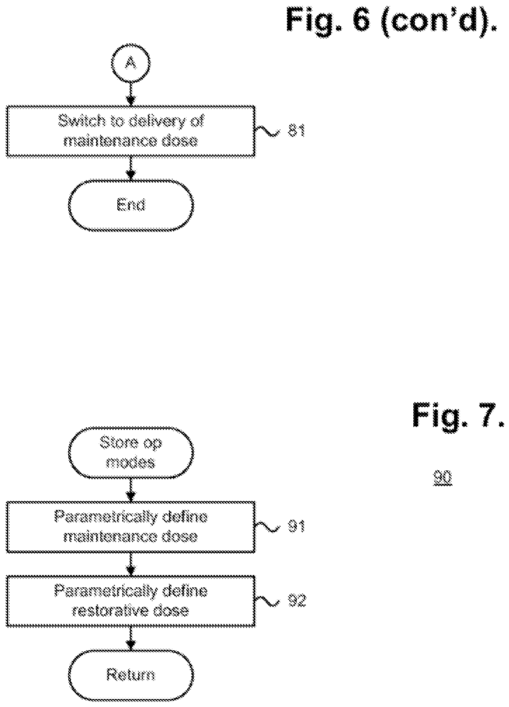

In one embodiment, the neural stimulation is provided in a low level maintenance dose independent of cardiac cycle and, upon sensing a condition indicative of spontaneous tachyarrhythmia, the stimulation device 11 delivers a restorative dose of VNS at a higher level of intensity than the maintenance dose. VNS maintenance dose delivery is only continually resumed if, during post-suspension monitoring, tachyarrhythmia is not found to recur. The stimulation device 11 bi-directionally stimulates the vagus nerve 15, 16 through multimodal application of continuously-cycling, intermittent and periodic electrical stimuli, which are parametrically defined through stored stimulation parameters and timing cycles, as further described infra with reference to FIGURE 7. In a further embodiment, bradycardia in VNS-titrated patients can be managed through suspension of on-going low-level VNS.

In another embodiment, the stimulation device 11 delivers VNS while the patient 10 is awake. The stimulation device 11 bi-directionally stimulates the vagus nerve 15, 16 through multimodal application of continuously-cycling, intermittent and periodic electrical stimuli, which are parametrically defined through stored stimulation parameters and timing cycles. Upon patient awakening, an enhanced dose of VNS is delivered to counter the increased tachyarrhythmic risk caused by the natural circadian rhythm-triggered surge of sympathetic activity and increased heart rate. In a further embodiment, tachyarrhythmias outside of awakening can be managed through application of a restorative dose of VNS upon the sensing of a condition indicative of tachyarrhythmias..

In another embodiment, during non-exertion periods, that is, periods when the patient 10 is neither actively exercising nor undergoing other physical exertion, and is also not recovering from exercise, the stimulation device 11 delivers VNS. The stimulation device 11 bi-directionally stimulates the vagus nerve 15, 16 through multimodal application of continuously-cycling, intermittent and periodic electrical stimuli, which are parametrically defined through stored stimulation parameters and timing cycles. Immediately following exercise during the post-exercise recovery period, an enhanced dose of VNS is delivered to ameliorate the increased tachyarrhythmic risk occasioned by elevated sympathetic activation and release of epinephrine (adrenaline) and norepinephrine (noradrenaline). In a further embodiment, non-exertion induced tachyarrhythmias can be managed through application of a restorative dose of VNS upon the sensing of a condition indicative of tachyarrhythmias..

Both sympathetic and parasympathetic nerve fibers are stimulated. Cervical vagus nerve stimulation results in propagation of action potentials from the site of stimulation in a manner that results in creation and propagation (in both afferent and efferent directions) of action potentials within neuronal fibers comprising the cervical vagus nerve to restore cardiac autonomic balance. Afferent action potentials propagate toward the parasympathetic nervous system's origin in the medulla in the nucleus ambiguus, nucleus tractus solitarius, and the dorsal motor nucleus, as well as towards the sympathetic nervous system's origin in the intermediolateral cell column of the spinal cord. Efferent action potentials propagate toward the heart 17 to activate the components of the heart's intrinsic nervous system. Either the left or right vagus nerve 15, 16 can be stimulated by the stimulation device 11. The right vagus nerve 16 has a moderately lower stimulation threshold than the left vagus nerve 15 for heart rate affects at the same parametric levels.

The VNS therapy is delivered autonomously to the patient's vagus nerve 15, 16 through three implanted components that include a neurostimulator 12, therapy lead 13, and helical electrodes 14. FIGURES 2A and 2B are diagrams respectively showing the implantable neurostimulator 12 and the stimulation therapy lead 13 of FIGURE 1. In one embodiment, the neurostimulator 12 can be adapted from a VNS Therapy AspireSR Model 106 pulse generator, manufactured and sold by Cyberonics, Inc., Houston, TX, although other manufactures and types of single-pin receptacle implantable VNS neurostimulators with integrated leadless heart rate sensors could also be used. The stimulation therapy lead 13 and helical electrodes 14 are generally fabricated as a combined assembly and can be adapted from a Model 302 lead, PerenniaDURA Model 303 lead, or PerenniaFLEX Model 304 lead, also manufactured and sold by Cyberonics, Inc., in two sizes based on helical electrode inner diameter, although other manufactures and types of single-pin receptacle-compatible therapy leads and electrodes could also be used.

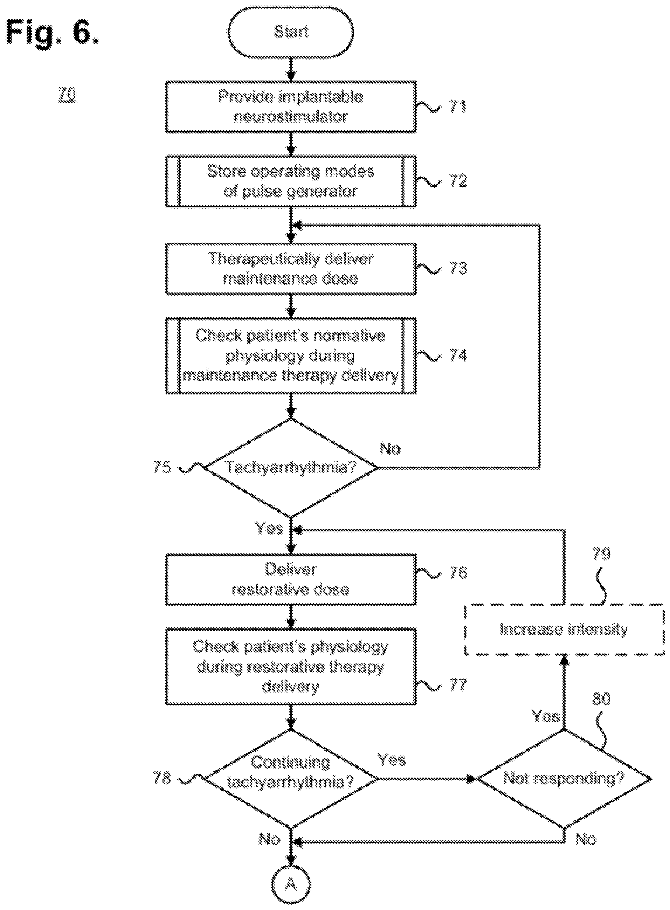

Referring first to FIGURE 2A, the neurostimulator 12 provides multimodal vagal stimulation. In a maintenance mode, the neurostimulator 12 is parametrically programmed to deliver continuously-cycling, intermittent and periodic ON-OFF cycles of VNS are delivered that produce action potentials in the underlying nerves that propagate bi-directionally. In a restorative mode, the neurostimulator 12 is parametrically programmed to deliver VNS tuned to prevent initiation of or disrupt tachyarrhythmia through continuously-cycling, intermittent and periodic ON-OFF cycles of VNS delivered at higher intensity, which could be higher output current, higher duty cycle, higher frequency, longer pulse width, or a combination of the foregoing parameters, than the maintenance dose are delivered in response to the onset or progression of tachyarrhythmias. In an enhanced mode, which is further described with reference to FIGURES 6 and 7, the neurostimulator 12 is parametrically programmed to deliver the VNS tuned to prevent initiation of or disrupt tachyarrhythmia specifically upon patient awakening through continuously-cycling, intermittent and periodic ON-OFF cycles of VNS is delivered at delivered at a higher intensity than the maintenance mode, higher output current, higher duty cycle, higher frequency, longer pulse width, or a combination of the foregoing parameters.

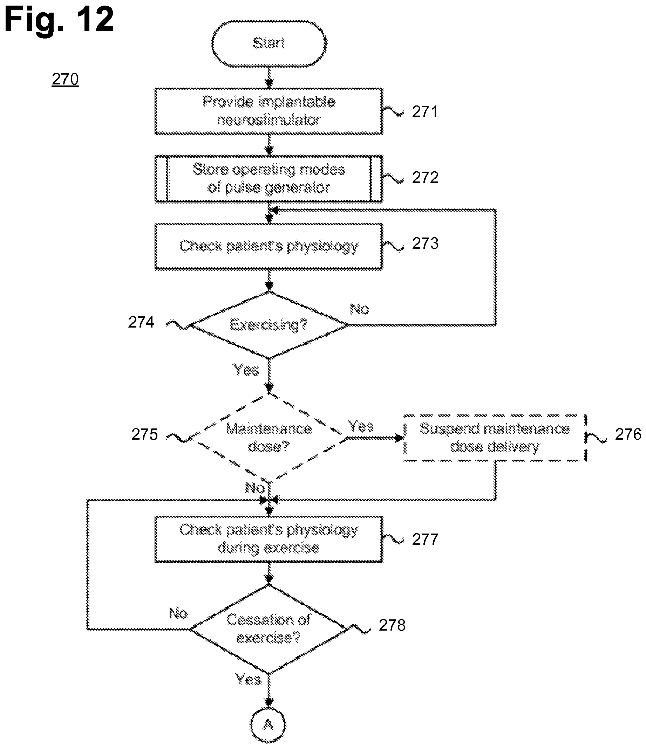

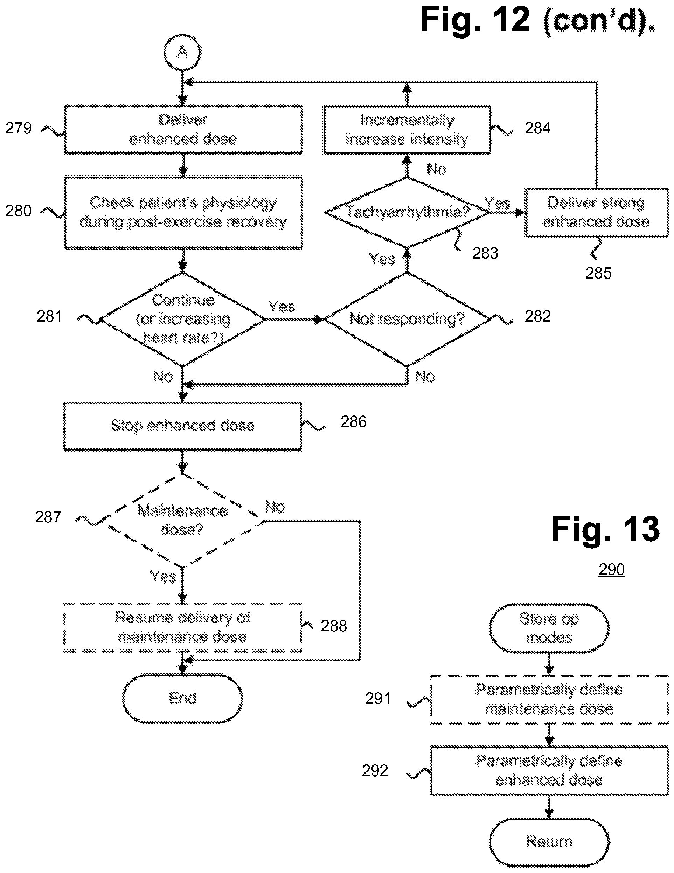

In another embodiment, during post-exercise recovery period, the neurostimulator 12 is parametrically programmed to deliver an enhanced dose of continuously-cycling, intermittent and periodic ON-OFF cycles of VNS, that is delivered to produce action potentials in the underlying nerves that propagate bi-directionally during non-exertion periods, as further described infra beginning with reference to FIGURE 12. The enhanced dose is tuned to prevent initiation of or disrupt tachyarrhythmia. In a further embodiment, the neurostimulator 12 is parametrically programmed to deliver a maintenance dose of continuously-cycling, intermittent and periodic ON-OFF cycles of VNS, that is delivered to produce action potentials in the underlying nerves that propagate bi-directionally. The maintenance dose is delivered at lower intensity, which could be lower output current, lower duty cycle, lower frequency, shorter pulse width, or a combination of the foregoing parameters, than the enhanced dose delivered during post-exercise recovery period.

The neurostimulator 12 includes an electrical pulse generator that is tuned to restore autonomic balance by triggering action potentials that propagate both afferently and efferently within the vagus nerve 15, 16. The neurostimulator 12 is enclosed in a hermetically sealed housing 21 constructed of a biocompatible, implantation-safe material, such as titanium. The housing 21 contains electronic circuitry 22 powered by a primary battery 23, such as a lithium carbon monoflouride battery. The electronic circuitry 22 is implemented using complementary metal oxide semiconductor integrated circuits that include a microprocessor controller that executes a control program according to stored stimulation parameters and timing cycles; a voltage regulator that regulates system power; logic and control circuitry, including a recordable memory 29 within which the stimulation parameters are stored, that controls overall pulse generator function, receives and implements programming commands from the external programmer, or other external source, collects and stores telemetry information, processes sensory input, and controls scheduled and sensory-based therapy outputs; a transceiver that remotely communicates with the external programmer using radio frequency signals; an antenna, which receives programming instructions and transmits the telemetry information to the external programmer; and a reed switch 30 that provides remote access to the operation of the neurostimulator 12 using an external programmer, a simple patient magnet, or an electromagnetic controller. The recordable memory 29 can include both volatile (dynamic) and persistent (static) forms of memory, such as firmware within which the stimulation parameters and timing cycles can be stored. Other electronic circuitry and components are possible.

Externally, the neurostimulator 12 includes a header 24 to securely receive and connect to the therapy lead 13. In one embodiment, the header 24 encloses a receptacle 25 into which a single pin for the therapy lead 13 can be received, although two or more receptacles could also be provided, along with the requisite additional electronic circuitry 22. The header 24 internally includes a lead connector block (not shown) and a set of set screws 26.

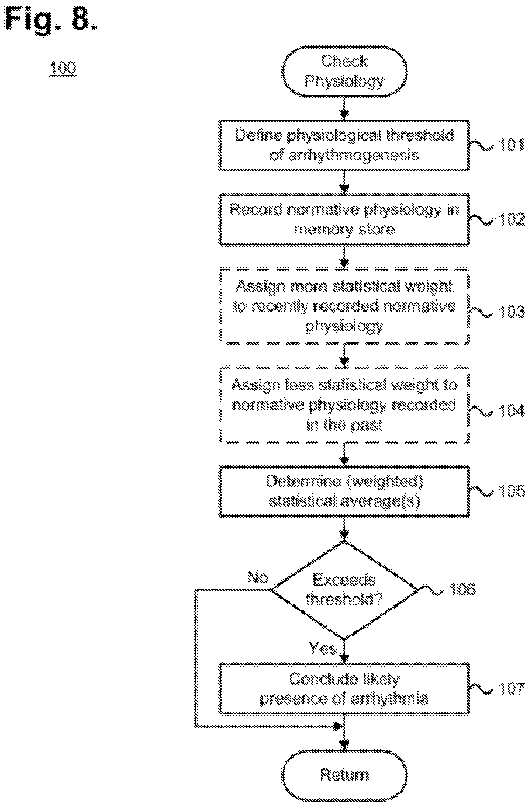

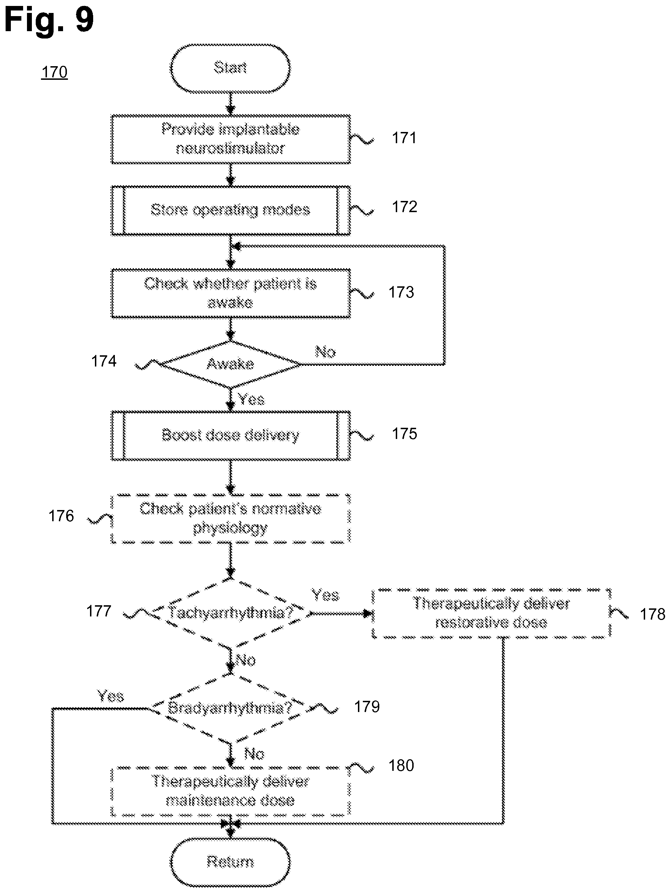

The housing 21 can also contain a heart rate sensor 31 that is electrically interfaced with the logic and control circuitry, which receives the patient's sensed heart rate as sensory inputs. The heart rate sensor 31 monitors heart rate using an ECG-type electrode. Through the electrode, the patient's heart beat can be sensed by detecting ventricular depolarization. In a further embodiment, a plurality of electrodes can be used to sense voltage differentials between electrode pairs, which can undergo signal processing for cardiac physiological measures, for instance, detection of the P-wave, QRS complex, and T-wave. The heart rate sensor 31 provides the sensed heart rate to the control and logic circuitry as sensory inputs that can be used to determine the onset or presence of arrhythmias, particularly VT, as further described infra with reference to FIGURE 8. in another embodiment, the heart rate sensor 31 provides the sensed heart rate to the control and logic circuitry as sensory inputs that can be used to monitor whether the patient 10 is awake or asleep, as further described infra with reference to FIGURE 9 and determine the presence of possible tachyarrhythmias, particularly VT. In another embodiment, the heart rate sensor 31 provides the sensed heart rate to the control and logic circuitry as sensory inputs that can be used to sense cessation of physical exercise and determine the presence of possible tachyarrhythmias, particularly VT, during post-exercise recovery period.

In a further embodiment, the housing 21 contains a minute ventilation sensor 32 that is electrically interfaced with the logic and control circuitry, which receives the patient's respiratory dynamics as sensory inputs. The minute ventilation sensor 32, such as described in U.S. Patent No. 7,092,757, issued August 15, 2006, to Larson et al., the patient's respiratory rate and tidal volume, and calculates the patient's minute ventilation volume. The minute ventilation sensor 32 provides the minute ventilation volume to the control and logic circuitry as sensory inputs that can be used to determine whether the patient is awake.

In a still further embodiment, the housing 21 contains an accelerometer 33 that is electrically interfaced with the logic and control circuitry, which receives the patient's physical movement as sensory inputs. The minute ventilation sensor 32 may be combined into a blended sensor with at least one accelerometer 33. The accelerometer 33 contains the circuitry and mechanical components necessary to measure acceleration of the patient's body along at least two axes, and may include multiple uniaxial accelerometers, a dual axial accelerometer, or a triaxial accelerometer. By measuring the acceleration along multiple axes, the accelerometer 33 provides sensory inputs that can be used to determine the patient's posture and rate of movement and whether the patient has fallen or awakened from sleep. In a further embodiment, the accelerometer 33 can be located separately from the minute ventilation sensor 32, either on the interior or exterior of the housing 21.

In some embodiments, by measuring the acceleration along multiple axes, the accelerometer 33 provides sensory inputs that can be used to determine the patient's posture and rate of movement, which can augment or supplant the heart rate sensor 31 in sensing cessation of physical exercise. In some embodiments, with respect to the minute ventilation sensor 32, the relationship between oxygen uptake and tidal volume during aerobic metabolism closely ties minute ventilation to heart rate during physical exercise, which can augment or supplant the heart rate sensor 31 and accelerometer 33 in sensing cessation of physical exercise.

The neurostimulator 12 is preferably interrogated prior to implantation and throughout the therapeutic period with a healthcare provider-operable external programmer and programming wand (not shown) for checking proper operation, downloading recorded data, diagnosing problems, and programming operational parameters, such as described in commonly-assigned U.S. Patent applications, Serial No. 13/314,130 and 13/352,244, cited supra. Generally, use of the external programmer is restricted to healthcare providers, while more limited manual control is provided to the patient through "magnet mode." In one embodiment, the external programmer executes application software specifically designed to interrogate the neurostimulator 12. The programming computer interfaces to the programming wand through a standardized wired or wireless data connection. The programming wand can be adapted from a Model 201 Programming Wand, manufactured and sold by Cyberonics, Inc. and the application software can be adapted from the Model 250 Programming Software suite, licensed by Cyberonics, Inc. Other configurations and combinations of external programmer, programming wand and application software are possible.

The neurostimulator 12 delivers VNS under control of the electronic circuitry 22. The stored stimulation parameters are programmable. Each stimulation parameter can be independently programmed to define the characteristics of the cycles of therapeutic stimulation and inhibition to ensure optimal stimulation for a patient 10. The programmable stimulation parameters include output current, signal frequency, pulse width, signal ON time, signal OFF time, magnet activation (for VNS specifically triggered by "magnet mode"), and reset parameters. Other programmable parameters are possible. In addition, sets or "profiles" of preselected stimulation parameters can be provided to physicians with the external programmer and fine-tuned to a patient's physiological requirements prior to being programmed into the neurostimulator 12, such as described in commonly-assigned U.S. Patent application, entitled "Computer-Implemented System and Method for Selecting Therapy Profiles of Electrical Stimulation of Cervical Vagus Nerves for Treatment of Chronic Cardiac Dysfunction," Serial No. 13/314,138, filed on December 7, 2011, pending, the disclosure of which is incorporated by reference.

Referring next to FIGURE 2B, the therapy lead 13 delivers an electrical signal from the neurostimulator 12 to the vagus nerve 15, 16 via the helical electrodes 14. On a proximal end, the therapy lead 13 has a lead connector 27 that transitions an insulated electrical lead body to a metal connector pin 28. During implantation, the connector pin 28 is guided through the receptacle 25 into the header 24 and securely fastened in place using the set screws 26 to electrically couple the therapy lead 13 to the neurostimulator 12. On a distal end, the therapy lead 13 terminates with the helical electrode 14, which bifurcates into a pair of anodic and cathodic electrodes. In one embodiment, the lead connector 27 is manufactured using silicone and the connector pin 28 is made of stainless steel, although other suitable materials could be used, as well. The insulated lead body 13 utilizes a silicone-insulated alloy conductor material.

Preferably, the helical electrodes 14 are placed over the cervical vagus nerve 15, 16 at the location below where the superior and inferior cardiac branches separate from the cervical vagus nerve. In alternative embodiments, the helical electrodes may be placed at a location above where one or both of the superior and inferior cardiac branches separate from the cervical vagus nerve. In one embodiment, the helical electrodes 14 are positioned around the patient's vagus nerve oriented with the end of the helical electrodes 14 facing the patient's head. In an alternate embodiment, the helical electrodes 14 are positioned around the patient's vagus nerve 15, 16 oriented with the end of the helical electrodes 14 facing the patient's heart 17. At the distal end, the insulated electrical lead body 13 is bifurcated into a pair of lead bodies that are connected to a pair of electrodes proper. The polarity of the electrodes can be configured into a monopolar cathode, a proximal anode and a distal cathode, or a proximal cathode and a distal anode.

Therapeutically, the VNS is delivered as a multimodal set of therapeutic and event-based doses, which are system output behaviors that are pre-specified within the neurostimulator 12 through the stored stimulation parameters and timing cycles implemented in firmware and executed by the microprocessor controller. The therapeutic doses include a cardiac cycle-independent maintenance dose that includes continuously-cycling, intermittent and periodic cycles of electrical stimulation during periods in which the pulse amplitude is greater than 0 mA ("therapy ON") and during periods in which the pulse amplitude is 0 mA ("therapy OFF"). The therapeutic doses also include a restorative dose at a higher level of intensity than the maintenance dose, which could be higher output current, higher duty cycle, higher frequency, longer pulse width, or a combination of the foregoing parameters, in response to the presence of tachyarrhythmias.

In some embodiments, the therapeutic doses also include an enhanced dose of VNS tuned to prevent initiation of or disrupt tachyarrhythmia upon awakening through continuously-cycling, intermittent and periodic ON-OFF cycles of VNS delivered at a higher intensity than the maintenance dose, which could be higher output current, higher duty cycle, higher frequency, longer pulse width, or a combination of the foregoing parameters, in response to the presence of tachyarrhythmias upon the patient's 10 awakening.

In some embodiments, the therapeutic doses include a cardiac cycle-independent enhanced dose delivered during post-exercise recovery period that includes continuously-cycling, intermittent and periodic cycles of electrical stimulation during periods in which the pulse amplitude is greater than 0 mA ("therapy ON") and during periods in which the pulse amplitude is 0 mA ("therapy OFF"). The therapeutic doses also include, in a further embodiment, a maintenance dose that is delivered at a lower level of intensity than the enhanced dose, which could be lower output current, lower duty cycle, lower frequency, shorter pulse width, or a combination of the foregoing parameters, during non-exertion periods.

The neurostimulator 12 can operate either with or without an integrated heart rate sensor (provided that patient physiology can be monitored through some other type of sensing mechanism), such as respectively described in commonly-assigned U.S. Patent application, entitled "Implantable Device for Providing Electrical Stimulation of Cervical Vagus Nerves for Treatment of Chronic Cardiac Dysfunction with Leadless Heart Rate Monitoring," Serial No. 13/314,126, filed on December 7, 2011, pending, and U.S. Patent application, entitled "Implantable Device for Providing Electrical Stimulation of Cervical Vagus Nerves for Treatment of Chronic Cardiac Dysfunction," Serial No. 13/314,119, filed on December 7, 2011, pending. Additionally, where an integrated leadless heart rate monitor is available, the neurostimulator 12 can provide autonomic cardiovascular drive evaluation and self-controlled titration, such as respectively described in commonly-assigned U.S. Patent application, entitled "Implantable Device for Evaluating Autonomic Cardiovascular Drive in a Patient Suffering from Chronic Cardiac Dysfunction," Serial No. 13/314,133, filed on December 7, 2011, pending, and U.S. Patent application, entitled "Implantable Device for Providing Electrical Stimulation of Cervical Vagus Nerves for Treatment of Chronic Cardiac Dysfunction with Bounded Titration," Serial No. 13/314,135, filed on December 7, 2011, pending. The neurostimulator 12 can be used to counter natural circadian sympathetic surge upon awakening and manage the risk of cardiac arrhythmias during or attendant to sleep, particularly sleep apneic episodes, and the neurostimulator 12 can be used to ameliorate heart rate increase and decrease tachyarrhythmic risk following exercise.

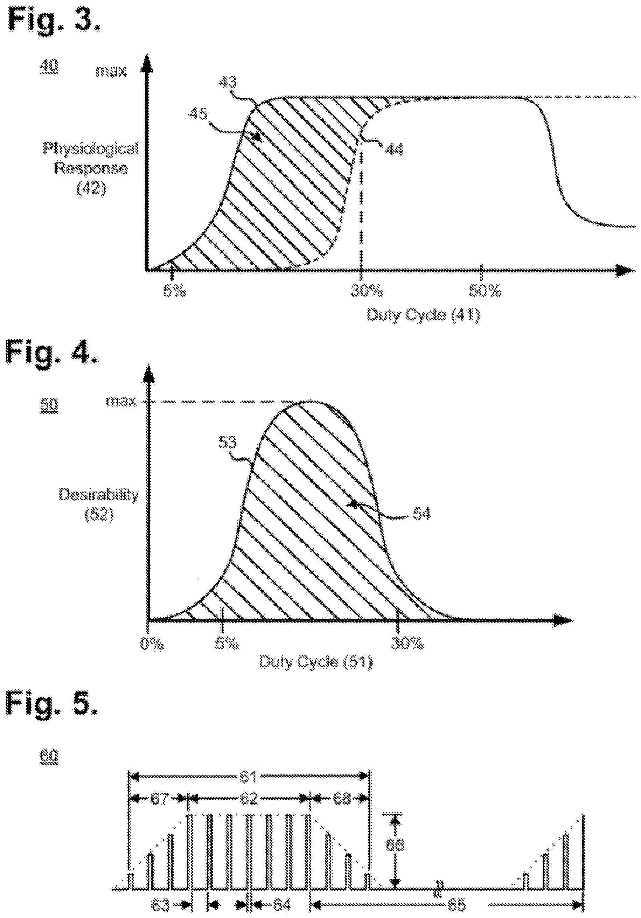

Therapeutically, VNS is delivered as a heart failure therapy, during the period following awakening, and for post-exercise recovery attenuation independent of cardiac cycle and in a maintenance or an enhanced dose having an intensity that is insufficient to elicit side-effects, such as cardiac arrhythmias. The VNS can be delivered with a periodic duty cycle in the range of 2% to 89% with a preferred range of around 4% to 36% that is delivered as a low intensity maintenance dose. The selection of duty cycle is a tradeoff among competing medical considerations. FIGURE 3 is a graph 40 showing, by way of example, the relationship between the targeted therapeutic efficacy 43 and the extent of potential side effects 44 resulting from use of the implantable neurostimulator 12 of FIGURE 1. The x-axis represents the duty cycle 41. The duty cycle is determined by dividing the stimulation ON time by the sum of the ON and OFF times of the neurostimulator 12 during a single ON-OFF cycle. However, the stimulation time may also need to include ramp-up time and ramp-down time, where the stimulation frequency exceeds a minimum threshold (as further described infra with reference to FIGURE 5). The y-axis represents physiological response 42 to VNS therapy. The physiological response 42 can be expressed quantitatively for a given duty cycle 41 as a function of the targeted therapeutic efficacy 43 and the extent of potential side effects 44, as described infra. The maximum level of physiological response 42 ("max") signifies the highest point of targeted therapeutic efficacy 43 or potential side effects 44.

Targeted therapeutic efficacy 43 and the extent of potential side effects 44 can be expressed as functions of duty cycle 41 and physiological response 42. The targeted therapeutic efficacy 43 represents the intended effectiveness of VNS in provoking a beneficial physiological response for a given duty cycle and can be quantified by assigning values to the various acute and chronic factors that contribute to the physiological response 42 of the patient 10 due to the delivery of therapeutic VNS. Acute factors that contribute to the targeted therapeutic efficacy 43 include beneficial changes in heart rate variability and increased coronary flow, reduction in cardiac workload through vasodilation, and improvement in left ventricular relaxation. Chronic factors that contribute to the targeted therapeutic efficacy 43 include improved cardiovascular regulatory function, as well as decreased negative cytokine production, increased baroreflex sensitivity, increased respiratory gas exchange efficiency, favorable gene expression, renin-angiotensin-aldosterone system down-regulation, anti-arrhythmic, anti-apoptotic, and ectopy-reducing anti-inflammatory effects. These contributing factors can be combined in any manner to express the relative level of targeted therapeutic efficacy 43, including weighting particular effects more heavily than others or applying statistical or numeric functions based directly on or derived from observed physiological changes. Empirically, targeted therapeutic efficacy 43 steeply increases beginning at around a 5% duty cycle, and levels off in a plateau near the maximum level of physiological response at around a 30% duty cycle. Thereafter, targeted therapeutic efficacy 43 begins decreasing at around a 50% duty cycle and continues in a plateau near a 25% physiological response through the maximum 100% duty cycle.

The intersection 45 of the targeted therapeutic efficacy 43 and the extent of potential side effects 44 represents one optimal duty cycle range for VNS. FIGURE 4 is a graph 50 showing, by way of example, the optimal duty cycle range 53 based on the intersection 45 depicted in FIGURE 3. The x-axis represents the duty cycle 51 as a percentage of stimulation time over inhibition time. The y-axis represents therapeutic points 52 reached in operating the neurostimulator 12 at a given duty cycle 51. The optimal duty range 53 is a function 54 of the intersection 44 of the targeted therapeutic efficacy 43 and the extent of potential side effects 44. The therapeutic operating points 52 can be expressed quantitatively for a given duty cycle 51 as a function of the values of the targeted therapeutic efficacy 43 and the extent of potential side effects 44 at their point of intersection in the graph 40 of FIGURE 3. The optimal therapeutic operating point 55 ("max") signifies a tradeoff that occurs at the point of highest targeted therapeutic efficacy 43 in light of lowest potential side effects 44 and that point will typically be found within the range of a 5% to 30% duty cycle 51. Other expressions of duty cycles and related factors are possible.

Therapeutically and in the absence of patient physiology of possible medical concern, such as cardiac arrhythmias, or following the patient's awakening from sleep, VNS is delivered in a low level maintenance dose that uses alternating cycles of stimuli application (ON) and stimuli inhibition (OFF) that are tuned to activate both afferent and efferent pathways. In some embodiments, VNS is delivered during non-exertion periods. Stimulation results in parasympathetic activation and sympathetic inhibition, both through centrally-mediated pathways and through efferent activation of preganglionic neurons and local circuit neurons. FIGURE 5 is a timing diagram showing, by way of example, a stimulation cycle and an inhibition cycle of VNS 60 as provided by implantable neurostimulator 12 of FIGURE 1. The stimulation parameters enable the electrical stimulation pulse output by the neurostimulator 12 to be varied by both amplitude (output current 66) and duration (pulse width 64). The number of output pulses delivered per second determines the signal frequency 63. In one embodiment, a pulse width in the range of 100 to 250 µsec delivers between 0.02 and 50 mA of output current at a signal frequency of about 20 Hz, although other therapeutic values could be used as appropriate.

In the simplest case, the stimulation time is the time period during which the neurostimulator 12 is ON and delivering pulses of stimulation. The OFF time 65 is always the time period occurring in-between stimulation times 61 during which the neurostimulator 12 is OFF and inhibited from delivering stimulation. In one embodiment, the neurostimulator 12 implements a ramp-up time 67 and a ramp-down time 68 that respectively precede and follow the ON time 62 during which the neurostimulator 12 is ON and delivering pulses of stimulation at the full output current 66. The ramp-up time 67 and ramp-down time 68 are used when the stimulation frequency is at least 10 Hz, although other minimum thresholds could be used, and both ramp-up and ramp-down times 67, 68 last two seconds, although other time periods could also be used. The ramp-up time 67 and ramp-down time 68 allow the strength of the output current 66 of each output pulse to be gradually increased and decreased, thereby avoiding deleterious reflex behavior due to sudden delivery or inhibition of stimulation at a programmed intensity.

The triggering of CHF compensatory mechanisms underlying a CCD increases the risk of tachyarrhythmias. Furthermore, in the hours following awakening and after physical exercise or other physical activity, the risk of tachyarrhythmia is even higher. Although delivered in a maintenance dose, or in a further embodiment, an enhanced dose upon patient awakening or, in a further embodiment, in a maintenance dose while the patient is awake, or, in a further embodiment, in an enhanced dose during post-recovery period, or, in a further embodiment, in a maintenance dose during non-exertion periods,, with an intensity that is insufficient to elicit side-effects, such as cardiac arrhythmias, therapeutic VNS can nevertheless potentially prevent formation of pathological tachyarrhythmias in some patients, or ameliorate their occurrence during awakening in some patients, or ameliorate their occurrence during post-exercise recovery period in some patients. Although VNS has been shown to decrease defibrillation threshold, VNS is unlikely to terminate VF in the absence of defibrillation. VNS prolongs ventricular action potential duration, so may be effective in terminating VT. In addition, the effect of VNS on the AV node may be beneficial in patients with AF by slowing conduction to the ventricles and controlling ventricular rate.

Therapeutic maintenance dose VNS therapy can be suspended upon the occurrence of tachyarrhythmia and replaced with the delivery of higher intensity VNS that is tuned to prevent initiation of or disrupt tachyarrhythmia, after which therapy only resumes if tachyarrhythmia does not recur. In an alternative embodiment, neither of the maintenance or restorative doses are appropriate for the increased period of risk that a patient experiences upon waking up. The maintenance dose may be too low to prevent the occurrence of tachyarrhythmia during the diurnal peak, while the restorative dose may subject the patient to a high dose of VNS needlessly on occasions that a tachyarrhythmic event does not occur. In an alternative embodiment, upon sensing cessation of physical exercise or other physical exertion, VNS that is tuned to prevent initiation of or disrupt tachyarrhythmia is provided in an enhanced dose during post-exercise recovery period for a fixed period of time or as determined by heart response trajectory.

FIGURE 6 is a flow diagram showing an implantable neurostimulator implemented method for managing tachyarrhythmias through vagus nerve stimulation 70, in accordance with one embodiment. The method is implemented on the stimulation device 11, the operation of which is parametrically defined through stored stimulation parameters and timing cycles.

Preliminarily, an implantable neurostimulator 12 with an integrated heart rate sensor 31, which includes a pulse generator 11, a nerve stimulation therapy lead 13, and a pair of helical electrodes 14, is provided (step 71). In an alternative embodiment, electrodes may be implanted with no implanted neurostimulator or leads. Power may be provided to the electrodes from an external power source and neurostimulator through wireless RF or inductive coupling. Such an embodiment may result in less surgical time and trauma to the patient. Furthermore, the integrated heart rate sensor 31 could be omitted in lieu of other types of sensing mechanisms for measuring the patient's physiology.

The pulse generator stores a set of operating modes (step 72) that parametrically defines both a low level maintenance dose and a high level restorative dose of the stimulation, as further described infra with reference to FIGURE 7. Therapeutic VNS, as parametrically defined by the maintenance dose operating mode, is delivered to at least one of the vagus nerve (step 73). The pulse generator 11 delivers electrical therapeutic stimulation to the cervical vagus nerve of a patient 10 in a manner that results in creation and propagation (in both afferent and efferent directions) of action potentials within neuronal fibers of either the left or right vagus nerve 15, 16 independent of cardiac cycle.

During maintenance dose therapy delivery, the patient's normative physiology, which is physiology during normal sinus rhythm, is checked for tachyarrhythmias (step 74), as further described infra with reference to FIGURES 7 and 8. In general, the onset or presence of pathological tachyarrhythmia can be determined by heart rate or normal sinus rhythm through an endocardial electrogram, as well as rhythm stability, onset characteristics, and similar rate and rhythm morphological indicators, as conventionally used in cardiac rhythm management devices, such as described in K. Ellenbogen et al., "Clinical Cardiac Pacing and Defibrillation," Ch. 3, pp. 68-126 (2d ed. 2000). QRS complex alone may be insufficient to identify a specific type of tachyarrhythmia. A heart rate in excess of 100 bpm, for instance, can indicate the onset of tachyarrhythmia. Notwithstanding, while a narrow QRS complex usually indicates SVT tachycardia, a wide QRS complex can indicate any form of tachycardia, including VT tachycardia, as well as SVT and sinus tachycardia, and other indicia of tachycardia or tachyarrhythmia may be necessary before concluding that a change in therapeutic maintenance dose delivery is appropriate. For example, a wide QRS complex combined with a heart rate between around 120 bpm to 250 bpm strongly indicates the presence of VT. In a further embodiment, the onset or presence of arrhythmias, particularly VT, can be determined with the assistance of a recency filter, as further described supra with reference to FIGURE 8. Still other physiological measures and indications of tachyarrhythmias are possible.

If a monitored physiological condition is indicative of tachyarrhythmia, that is, the patient's physiology indicates the onset or presence of tachyarrhythmia (step 75), the delivery of the maintenance dose is suspended and replaced with the delivery of a restorative dose of higher intensity VNS that is tuned to prevent initiation of or disrupt tachyarrhythmia (step 76). The patient's physiology is periodically monitored during the delivery of the restorative dose (step 77), the delivery of which is maintained (steps 76-77) while the tachyarrhythmia condition continues (step 78). If, after multiple checks of the patient's physiology (step 77), the arrhythmia is not responding to the delivery of the restorative dose (step 76), absent an improvement in heart rhythm, such as a decrease in the rate of the arrhythmia (step 77), restorative dose delivery is discontinued and treatment reverts to delivering the maintenance dose (step 81) after a set period of time following the termination of the arrhythmia (step 78).

In a further embodiment, delivery of the restorative dose can be manually suspended by providing the neurostimulator 12 with a magnetically-actuated reed switch that suspends delivery of the maintenance dose and resume delivery of the restorative dose, such as when the maintenance dose is tolerable to the patient 10, while the restorative dose is intolerable.

In a still further embodiment, the intensity of the restorative dose can be increased as necessary (step 80). For non-life-threatening or non-paroxysmal tachyarrhythmias, the intensity of the restorative dose is progressively increased, while for life-threatening or paroxysmal arrhythmias, a strong restorative dose of significantly higher intensity is used right away, due to the lack of time to ramp up the intensity progressively.

The recordable memory 29 in the electronic circuitry 22 of the neurostimulator 12 (shown in FIGURE 2A) stores the stimulation parameters that control the overall functionality of the pulse generator 11 in providing VNS therapy. FIGURE 7 is a flow diagram showing a routine 90 for storing operating modes for use with the method 70 of FIGURE 6. Two operating modes are stored, which include a maintenance dose of VNS tuned to restore cardiac autonomic balance (step 91) through continuously-cycling, intermittent and periodic electrical pulses and a restorative dose tuned to prevent initiation of or disrupt tachyarrhythmia (step 92) through periodic electrical pulses delivered at higher intensity than the maintenance dose.

In one embodiment, the autonomic regulation therapy is provided in a low level maintenance dose independent of cardiac cycle to activate both parasympathetic afferent and efferent nerve fibers in the vagus nerve simultaneously and a high level restorative dose. In the maintenance dose, a pulse width in the range of 250 to 500 µsec delivering between 0.02 and 1.0 mA of output current at a signal frequency in the range of 10 to 20 Hz, and a duty cycle of 5 to 30%, although other therapeutic values could be used as appropriate.

Different restorative doses are provided in response to different tachyarrhythmias. The restorative dose settings are physician-programmable. For a default restorative dose, the stimulation parameters would be in the same range as the maintenance dose, but would be moderately higher, with a pulse width again in the range of 250 to 500 µsec delivering between 1.5 and 2.0 mA of output current at a signal frequency in the range of 10 to 20 Hz. The duty cycle may change significantly from nominally 10% to temporarily 50% or 100%, although other therapeutic values could be used as appropriate. In addition, for non-life-threatening or non-paroxysmal tachyarrhythmias, the intensity of the restorative dose is progressively increased over time by increasing output current, duty cycle, or frequency, lengthening pulse width, or through a combination of the foregoing parameters. As well, discretely-defined restorative doses, each using different sets of parameters, may be delivered in the course of treating a single continuing tachyarrhythmic event, such as for life-threatening or paroxysmal arrhythmias that rapidly generate and require a significantly stronger restorative dose with no ramp up time.

The physiology of the patient 10 is monitored during the delivery of the maintenance dose and while undertaking rehabilitative measures, such as restorative dose delivery to counter an occurrence of arrhythmia, particularly VT, or other significant increase, decrease, or entrainment of cardiac rhythm. FIGURE 8 is a flow diagram showing an optional routine 100 for determining an onset or presence of arrhythmias with a recency filter for use with the method 70 of FIGURE 6. Normative physiology refers to the relative physiology of a patient suffering from CHF or other form of chronic cardiac dysfunction and the physiology of a particular CHF patient may vary from the norm as observed in a healthy non-CHF individual. Physiological thresholds of arrhythmogenesis are defined (step 101) that are used to establish conditions that indicate a departure from normal sinus rhythm. Periodically, the normative physiology of the patient 10 is recorded in the recordable memory 29 (shown in FIGURE 2A) (step 102). The normative physiology can include heart rate or normal sinus rhythm, as sensed by a physiological sensor, such as a heart rate monitor 31, as well as other available physiological data, for instance, as derivable from an endocardial electrogram. In a further embodiment, statistics can be stored in the recordable memory 29 for storage efficiency, instead of the raw sensed heart rate data. For instance, a binned average heart rate could be stored as representative of the patient's overall heart rate during a fixed time period. Based on the recorded normative physiology, a statistical average can be determined (step 105). If the statistical average exceeds the arrhythmogenic threshold (step 106), arrhythmia likely is present (step 107) and modification of the operating mode of the pulse generator 11 is indicated. The arrhythmia can include various forms of tachyarrhythmia, including atrial fibrillation (AF), ventricular tachyarrhythmias (ventricular tachycardia (VT) and ventricular fibrillation (VF)), and atrial flutter, and bradyarrhythmia, including atrial bradycardia, atrioventricular bradycardia, and ventricular bradycardia.

In a further embodiment, a form of recency filter is used to statistically favor recent events. By way of example, more statistical weight can be assigned to recently recorded normative physiology (step 103) and less statistical weight can be assigned to normative physiology recorded in the past (step 104). The lower weighting of older physiology values helps form a long-term running average that can be treated as a trailing baseline. A sliding window of physiology values collected, for example, over the past week can be applied to typify current patient condition. Weighted statistical averages are determined for recent and past normative physiology (step 105) and recent (foreground) and past (background or trailing) physiology averages can be compared (step 106) to identify arrhythmogenesis or other consideration.

In a still further embodiment, the sensed heart rate data can be used to analyze therapeutic efficacy and patient condition. For instance, statistics could be determined from the sensed heart rate, either onboard by the neurostimulator 12 or by an external device, such as a programming computer following telemetric data retrieval. The sensed heart rate data statistics can include determining a minimum heart rate over a stated time period, a maximum heart rate over a stated time period, an average heart rate over a stated time period, and a variability of heart rate over a stated period, where the stated period could be a minute, hour, day, week, month, or other selected time interval. Still other uses of the heart rate sensor 31 and the sensed heart rate data are possible

In still further embodiments, the suspension and resumption of either or both the delivery of the maintenance dose and the restorative dose can be titrated to gradually withdraw or introduce their respective forms of VNS. As well, both forms of VNS therapy delivery can be manually suspended by providing the neurostimulator 12 with a magnetically-actuated reed switch that suspends delivery of the maintenance dose and the restorative dose, as applicable, in response to a remotely applied magnetic signal.

FIGURE 9 is a flow diagram showing an implantable neurostimulator-implemented method for managing tachyarrhythmias upon the patient's 10 awakening through vagus nerve stimulation 170, in accordance with one embodiment. The method 170 is implemented on the stimulation device 11, the operation of which is parametrically defined through stored stimulation parameters and timing cycles. The method 170 can be used for treatment of both CHF and non-CHF patients suffering from other forms of chronic cardiac dysfunction.

Preliminarily, an implantable neurostimulator 12 with an integrated heart rate sensor 31, which includes a pulse generator 11, a nerve stimulation therapy lead 13, and a pair of helical electrodes 14, is provided (step 171). In an alternative embodiment, electrodes may be implanted with no implanted neurostimulator or leads. Power may be provided to the electrodes from an external power source and neurostimulator through wireless RF or inductive coupling. Such an embodiment may result in less surgical time and trauma to the patient. In a further embodiment, the integrated heart rate sensor 31 could be omitted in lieu of other types of sensing mechanisms for measuring the patient's physiology.

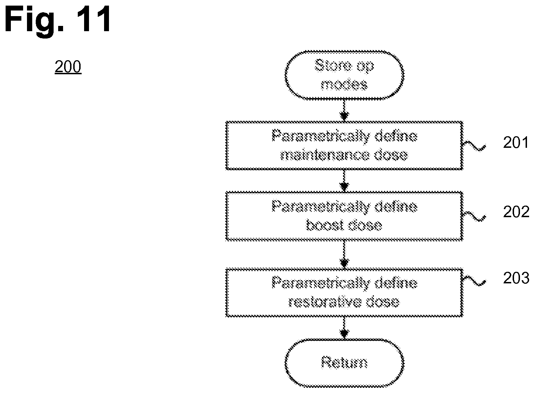

The pulse generator stores a set of one or more operating modes (step 172) that parametrically defines a low level maintenance dose, an enhanced dose, and a restorative dose of VNS stimulation, the latter two of which are higher in intensity than the maintenance dose, as further described infra with reference to FIGURE 11. A sleeping patient's 10 physiological state is regularly monitored to determine whether the patient 10 has awakened from sleep (step 173). In a further embodiment, the risk of cardiac arrhythmias during or attendant to sleep, particularly sleep apneic episodes, can be managed by the implantable neurostimulator 12, such as described in commonly-assigned U.S. Patent Application, entitled "Implantable Neurostimulator-Implemented Method For Managing Tachyarrhythmic Risk During Sleep Through Vagus Nerve Stimulation," Serial No. 13/828,486, filed March 14, 2013, pending. In one embodiment, heart rate is used to check the patient's physiology using the heart rate sensor 31. A normative heart rate during sleep is generally considered to fall between 60 to 70 beats per minute (bpm). Upon awakening, the heart rate naturally rises to an awake range generally somewhere under 100 bpm, depending upon patient condition. The normative heart rate of the patient 10 is monitored and recorded periodically while asleep to determine whether the patient 10 is awakening.

In general, awakening is characterized by the gradual onset of an increased heart rate, which can be sensed by the neurostimulator 12, as well as by evaluation of rhythm stability or related rate and rhythm morphological indicators, such as conventionally used in cardiac rhythm management devices. If the heart rate of the patient 10 is gradually elevated above the mean normative heart rate level recorded during sleep, for instance, a heart rate that gradually increases over a seven-minute period and is then maintained for a non-transitory period of time, the patient 10 is considered to be awakening. In contrast, abrupt onset of increased heart rate could be indicative of a non-sinus tachyarrhythmia.

In a still further embodiment, a minute ventilation sensor 32 can be used to determine patient awakening. Minute ventilation is closely tied to heart rate during sleep, as ventilatory volume (tidal volume) and breathing frequency (respiratory rate) decrease synchronously, as does heart rate, as the patient falls asleep, then settles into a regular pattern. Tidal volume at rest is measured by the minute ventilation sensor 32. In general, tidal volume at rest is around 0.5 L/min and can increase up to 3 L/min at a higher intensity level of exertion. Similarly, respiratory rate at rest is measured by the minute ventilation 32. In general, respiratory rate at rest is around 12 to 16 breathes/min and can increase 40 to 50 breathes/min during maximum levels of activity. A normative activity level while asleep is established by determining means of the tidal volume and respiratory rate. If tidal volume and respiratory rate of the patient 10 respectively exceed the mean resting values of tidal volume and respiratory rate, the patient 10 is considered to be awakening. In a still further embodiment, the heart rate sensor 31 and the accelerometer 32 can be used in combination with the minute ventilation sensor 32. Still other measures and indications of awakening are possible.

In a still further embodiment, the neurostimulator 12 can use a multiple forms of sensory data in determining whether the patient 10 has awakened. As well, the neurostimulator 12 can assign more weight to one type of sensory data over other types of sensory data. For example, more weight can be assigned to accelerometer 33 data, which would discount a rise in heart rate that occurs while the patient 10 remains recumbent and otherwise still. Other ways of preferentially weighting the data are possible.

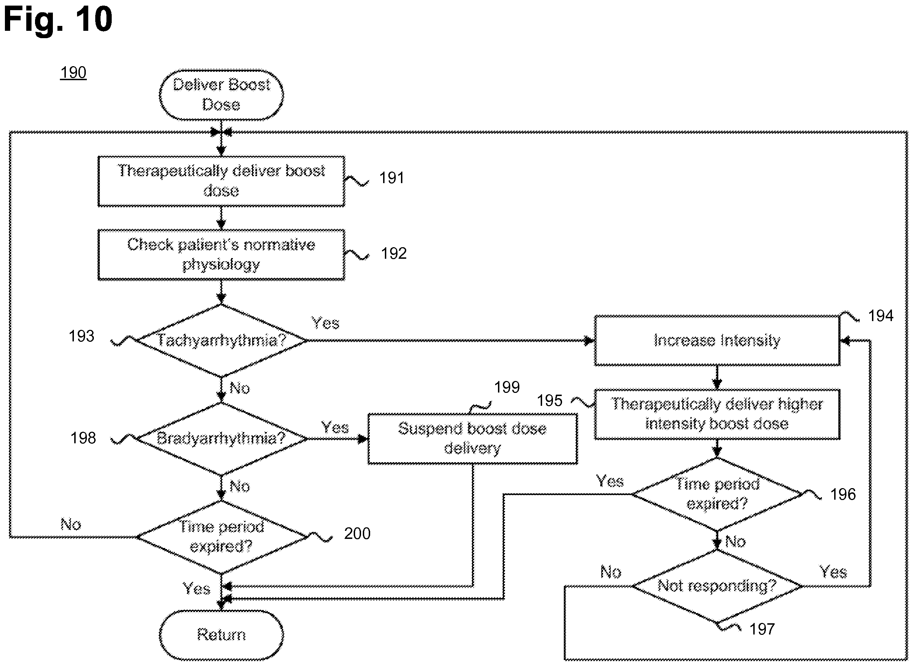

If the physiological state indicates that the patient 10 has not awakened from sleep (step 174), the patient's state is checked again periodically. If the monitored physiological state is indicative of the patient 10 having awakened (step 174), therapeutic VNS, as parametrically defined by the enhanced dose operating mode, is delivered to at least one of the vagus nerves through continuously-cycling, intermittent and periodic electrical pulses to tuned to prevent initiation of or disrupt tachyarrhythmia upon the patient's awakening (step 175), as further described infra with reference to FIGURE 10.

Following a completion of the enhanced dose delivery (step 175), a set of optional follow-up stimulation doses can be delivered as follows. The patient's normative physiology is monitored (step 176). If a condition indicative of tachyarrhythmia is present (step 177), a restorative dose of VNS stimulation is initiated (step 178), such as further described above. Contrarily, in the absence of tachyarrhythmia, the presence of bradyarrhythmia is assessed (step 179). If a condition indicative of bradyarrhythmia is detected (step 179), the method 170 is terminated and the bradyarrhythmia is addressed, such as described in commonly-assigned U.S. Patent Application, entitled "Implantable Neurostimulator-Implemented Method for Managing Bradycardia through Vagus Nerve Stimulation," Serial No. 13/544,656, filed on July 20, 2012, pending. If a condition indicative of a bradyarrhythmia is absent (step 179), a maintenance dose of VNS stimulation is initiated (step 180), such as further described above.

The enhanced dose of VNS stimulation helps ameliorate tachyarrhythmia vulnerability during awakening. FIGURE 10 is a flow diagram showing the routine 190 for providing an enhanced dose that is engaged upon the patient's 10 awakening for use in the method 170 of FIGURE 9. An enhanced dose, which has a higher intensity than the maintenance dose, is therapeutically delivered (step 191). In one embodiment, the enhanced dose is parametrically defined with a pulse width in the range of 250 to 500 µsec, delivering between 1.0 and 1.5 mA of output current at a signal frequency in the range of 10 to 20 Hz. The duty cycle may change significantly from nominally 10% to temporarily 50% or 100%, although other therapeutic values could be used as appropriate.

The patient's 10 normative physiology is monitored during delivery of the enhanced dose (step 192). The enhanced dose ameliorates, but does not fully eliminate, the risk of tachyarrhythmias. In general, the onset or presence of pathological tachyarrhythmia can be determined by heart rate or rhythm, as well as rhythm stability, onset characteristics, and similar rate and rhythm morphological indicators, as conventionally detected in cardiac rhythm management devices, such as described in K. Ellenbogen et al., "Clinical Cardiac Pacing and Defibrillation," Ch. 3, pp. 68-126 (2d ed. 2000). If a condition indicative of tachyarrhythmia is detected (step 193), the intensity of the enhanced dose is progressively increased (step 194) and delivered (step 195). The neurostimulator 12 checks whether the time period for the delivery of the enhanced dose has expired (step 196), terminating the routine 190 upon the period's expiration. In one embodiment, a fixed period of one to three hours is used, although the time period can be adjusted by a physician. In a further embodiment, the time period can be extended if a tachyarrhythmic condition occurs.

If the time period has not expired (step 196), the responsiveness of the tachyarrhythmia to the enhanced dose is assessed (step 197). Non-responsiveness to the delivery of VNS stimulation can occur during continuing heart rate elevation, which can present as no appreciable change in heart rate, insufficient heart rate decrease, or non-transitory increase in heart rate. Depending on the patient's 10 heart response trajectory, the intensity of the enhanced dose can be progressively increased by the same or similar amount each cycle (steps 194-197), or, for life-threatening or paroxysmal arrhythmias, immediately increased to a strongly enhanced dose of significantly higher intensity (step 194), due to the lack of time to ramp up the intensity progressively. The amount by which the intensity of the enhanced dose is progressively increased can also depend on the heart rate trajectory. In one embodiment, the strongly enhanced dose delivery (step 194) maximizes the VNS stimulation, delivering the maximum intensity of stimulation that the neurostimulator 12 can produce.

If the tachyarrhythmia is responding to the enhanced dose delivery (step 197), therapeutic delivery of the enhanced dose at default intensity is resumed (step 191) upon the termination of the tachyarrhythmia. In a further embodiment, the intensity of the enhanced dose can be increased continuously, independently of the heart response trajectory, for the duration of the period of time.

On the other hand, as the delivery of the enhanced dose is both preventative and precautionary, heart rate could decrease in response to the enhanced dose delivery as an unintended side-effect. If a condition indicative of bradyarrhythmia is detected (step 198), the enhanced dose delivery is suspended (step 199), terminating the routine 190.

Finally, in the absence of tachyarrhythmia (step 193) or bradyarrhythmia (step 198), the neurostimulator 12 checks whether the time period for delivery of the enhanced dose has expired (step 200), terminating the routine 190 if the period has expired. Otherwise, enhanced dose delivery continues (step 191).

In a still further embodiment, delivery of the enhanced, as well as the restorative dose, can be manually triggered, increased, decreased, or suspended by providing the neurostimulator 12 with a magnetically-actuated reed switch, such as described in commonly-assigned U.S. Patent applications, Serial Nos. 13/314,130 and 13/352,244, cited supra. In addition, the delivery of the enhanced dose and the maintenance dose can also be manually swapped. For instance, the switch can be used when the maintenance dose is tolerable to the patient 10, while the enhanced dose and the restorative dose are intolerable. Other uses of the switch are possible.

The recordable memory 29 in the electronic circuitry 22 of the neurostimulator 12 (shown in FIGURE 2A) stores the stimulation parameters that control the overall functionality of the pulse generator 11 in providing VNS therapy. FIGURE 11 is a flow diagram showing a routine 210 for storing operating modes for use with the method 170 of FIGURE 9. Three operating modes are stored, which include a maintenance dose of VNS tuned to restore cardiac autonomic balance (step 211) through continuously-cycling, intermittent and periodic electrical pulses; an enhanced dose of VNS tuned to prevent initiation of or disrupt tachyarrhythmia upon awakening through continuously-cycling, intermittent and periodic ON-OFF cycles of VNS delivered at a higher intensity than the maintenance dose (step 212); and a restorative dose tuned to prevent initiation of or disrupt tachyarrhythmia (step 213) through periodic electrical pulses delivered at a higher intensity than the maintenance dose.

In one embodiment, the autonomic regulation therapy is provided in a low level maintenance dose independent of cardiac cycle to activate both parasympathetic afferent and efferent neuronal fibers in the vagus nerve simultaneously and a high level enhanced dose. In the maintenance dose, a pulse width in the range of 250 to 500 µsec delivering between 0.02 and 1.0 mA of output current at a signal frequency in the range of 10 to 20 Hz, and a duty cycle of 5 to 30%, although other therapeutic values could be used as appropriate.

Different enhanced doses can be provided to respond to different tachyarrhythmic events. The enhanced dose settings are physician-programmable. For a default enhanced dose, the stimulation parameters would be in the same range as the maintenance dose, but would be moderately higher, with a pulse width again in the range of 250 to 500 µsec delivering between 1.5 and 2.0 mA of output current at a signal frequency in the range of 10 to 20 Hz. The duty cycle may change significantly from nominally 10% to temporarily 50% or 100%, although other therapeutic values could be used as appropriate. For non-life-threatening or non-paroxysmal tachyarrhythmias, the intensity of the enhanced dose is progressively increased over time by increasing output current, duty cycle, or frequency, lengthening pulse width, or through a combination of the foregoing parameters. Discretely-defined enhanced doses, each using different parameters sets, may be delivered in the course of treating a single continuing tachyarrhythmic event, such as for life-threatening or paroxysmal arrhythmias that rapidly generate and require a significantly strongly enhanced dose with no ramp up time.

In a further embodiment, the suspension and resumption of the enhanced dose, maintenance dose, or restorative dose can be titrated to gradually withdraw or introduce their respective forms of VNS.

FIGURE 12 is a flow diagram showing an implantable neurostimulator-implemented method for managing exercise-induced tachyarrhythmias through vagus nerve stimulation 270, in accordance with one embodiment. The method is implemented on the stimulation device 11, the operation of which is parametrically defined through stored stimulation parameters and timing cycles.

Preliminarily, an implantable neurostimulator 12 with an integrated heart rate sensor 31, which includes a pulse generator 11, a nerve stimulation therapy lead 13, and a pair of helical electrodes 14, is provided (step 271). In an alternative embodiment, electrodes may be implanted with no implanted neurostimulator or leads. Power may be provided to the electrodes from an external power source and neurostimulator through wireless RF or inductive coupling. Such an embodiment may result in less surgical time and trauma to the patient. Furthermore, the integrated heart rate sensor 31 could be omitted in lieu of or supplemented by other types of sensing mechanisms for measuring the patient's activity level and physiology, including an accelerometer 32 or minute ventilation sensor 33, as further described infra.

The pulse generator stores a set of one or more operating modes (step 272) that parametrically defines an enhanced dose and, in a further embodiment, a maintenance dose of the stimulation, as further described infra with reference to FIGURE 13. Patient's physiology is periodically checked (step 273). In one embodiment, heart rate is used to check the patient 10's physiology using the heart rate sensor 31. A normative heart rate is generally considered to fall between 60 to 100 beats per minute (bpm). When exercising, the heart rate may go up to 150 bpm or more, depending upon patient condition and degree of exertion. The normative heart rate of the patient 10 is monitored and recorded periodically during non-exertion periods to determine whether the patient 10 is now exercising or performing other types of physical exertion.

In general, engaging in physical exercise is characterized by the gradual onset of an elevated heart rate, as well as by evaluation of rhythm stability or related rate and rhythm morphological indicators, such as conventionally used in cardiac rhythm management devices. If the heart rate of the patient 10 is gradually elevated above the mean normative heart rate level, for instance, a heart rate that gradually increases to over 100 bpm over a five-minute period and is then maintained for a non-transitory period of time, the patient 10 is considered to be exercising. In contrast, abrupt onset of increased heart rate could be indicative of a non-sinus tachyarrhythmia.

In a further embodiment, an accelerometer 32 can be used to determine whether the patient's movement is indicative of exercise. The rate of change in patient's posture and movement are sensed by the accelerometer 32 during both non-exertion and exercise periods. A normative activity level is established by determining the mean of the frequency of movement during non-exertion periods. Both frequency and amplitude signals are continually sensed; increased frequency of movement is indicative of physical exertion. If the acceleration of the patient's physical movement exceeds the mean frequency of movement at the normative activity level, the patient is considered to be engaging in physical exercise. In a still further embodiment, the heart rate sensor 31, the accelerometer 32, or both can be used in combination.

In a still further embodiment, a minute ventilation sensor 33 can be used to determine a state of physical exercise. Minute ventilation is closely tied to heart rate during exercise, as ventilatory volume (tidal volume) and breathing frequency (respiratory rate) increase synchronously, as does heart rate, at a higher exercise level. Tidal volume at rest is measured by the minute ventilation sensor 33. In general, tidal volume at rest is around 0.5 L/min and can increase up to 3 L/min at a higher intensity level of exertion. Similarly, respiratory rate at rest is measured by the minute ventilation 33. In general, respiratory rate at rest is around 12 to 16 breathes/min and can increase 40 to 50 breathes/min during maximum levels of exercise. A normative activity level is established by determining means of the tidal volume and respiratory rate during non-exertion periods. If tidal volume and respiratory rate of the patient 10 respectively exceed the mean resting values of tidal volume and respiratory rate, the patient 10 is considered to be engaging in physical exercise. In a still further embodiment, the heart rate sensor 31 and the accelerometer 32 can be used in combination with the minute ventilation sensor 33. Still other measures and indications of engagement, as well as cessation, of physical exercise are possible.

In a still further embodiment, the neurostimulator 12 can use a multiple forms of sensory data in determining whether the patient 10 a state of physical exercise. As well, the neurostimulator 12 can assign more weight to one type of sensory data over other types of sensory data. For example, more weight can be assigned to accelerometer 32 data, which would discount a rise in heart rate that occurs while the patient 10 remains still, such as while seated and watching an exciting movie. Other ways of preferentially weighting the data are possible.

If the physiology indicates that the patient is exercising (step 274), an exercise protocol (steps 276-278) is initiated. If the patient 10 is receiving a maintenance dose (step 275), such as described above, the maintenance dose delivery is suspended (step 276). The maintenance dose is tuned to rehabilitatively restore cardiac autonomic balance through continuously-cycling, intermittent and periodic electrical pulses. However, in the context of continuous physical exercise, the continued delivery of the maintenance dose can potentially be counter-productive by influencing a decrease in heart rate during a time when the obverse affect on heart rate is desired.

During exercises, the patient's physiology is periodically checked to determine whether the patient 10 continues to exercise or has stopped (step 277). In one embodiment, cessation of physical exercise can be determined when a sustained heart rate of around 100 bpm or higher drops progressively, for instance, by at least 10 bpm. In general, a constant decrease in heart rate for more than three minutes indicates a cessation of physical exercise. In a further embodiment, when the increased frequency level of movement of the patient 10 measured by the accelerometer 32 drops and returns to the mean frequency of movement at the normative activity level, the data can indicate the cessation of physical exercise. In a still further embodiment, if tidal volume and respiratory rate of the patient 10 monitored by the minute ventilation sensor 33 gradually decrease, the data indicates that the patient 10 ceases from exercising.

Upon sensing cessation of physical exercise (step 278), a post-exercise recovery protocol (steps 279-285) is initiated. VNS, as parametrically defined by an enhanced dose in an operating mode, is delivered to at least one of the vagus nerve during post-exercise recovery period (step 279). The pulse generator 11 delivers electrical therapeutic stimulation to the cervical vagus nerve of the patient 10 in a manner that results in creation and propagation (in both afferent and efferent directions) of action potentials within neuronal fibers of either the left or right vagus nerve 15, 16 independent of cardiac cycle.

The patient's physiology is periodically monitored during the post-exercise recovery period (step 280), as described supra. Enhanced dose therapy delivery is continued for a fixed amount of time or, in a further embodiment, as determined by the patient's heart response trajectory based upon heart rate or sinus rhythm. If the fixed amount of time has not elapsed (step 281), the neurostimulator 12 continues the delivery of the enhanced dose (step 279).

In a further embodiment, rather than the fixed amount of time, the delivery of the enhanced dose is adapted to respond to the patient's observed heart response trajectory (step 281). During post-exercise recovery, the patient's heart rate is expected to continually decrease at a steady rate of about 17 bpm. In a patient suffering CCD, already elevated parasympathetic activation is exacerbated by the normally benign sinus tachyarrhythmia induced through exercise, which puts the patient 10 at risk of degenerate tachyarrhythmias, potentially VT and VF. The heart response trajectory during enhanced dose delivery is monitored (step 281) to evaluate heart rate responsiveness (step 282). Non-responsiveness to the delivery of the enhanced dose can occur due to continuing heart rate elevation, which can present as no appreciable change in heart rate, insufficient heart rate decrease, or non-transitory increase in heart rate. If the heart rate increase is significant, say, in excess of 180 bpm or more, the patient 10 may be suffering onset of a tachyarrhythmia (step 283) and a strongly enhanced dose of higher intensity VNS that is tuned to prevent initiation of or disrupt tachyarrhythmia is delivered (step 285). In general, the onset or presence of pathological tachyarrhythmia can be determined by heart rate or rhythm, as well as rhythm stability, onset characteristics, and similar rate and rhythm morphological indicators, as conventionally detected in cardiac rhythm management devices, such as described in K. Ellenbogen et al., "Clinical Cardiac Pacing and Defibrillation," Ch. 3, pp. 68-126 (2d ed. 2000). Otherwise, in the absence of tachyarrhythmia but continued non-responsiveness (step 283), the intensity of the enhanced dose may be incrementally increased (step 284) until improved response is seen or a maximum VNS dose is reached.

The delivery of the enhanced dose is maintained (steps 279-285). If, after multiple checks of the patient's physiology, the patient's physiology indicates improvement, such as satisfactory decrease in heart rate or having reached normal sinus rhythm, the enhanced dose is stopped (step 286). In a further embodiment, when the patient is receiving a maintenance dose prior to the physical exercise (step 287), the maintenance dose delivery is resumed (step 288).

In a still further embodiment, delivery of the enhanced dose, as well as the strongly enhanced dose, can be manually triggered, increased, decreased, or suspended by providing the neurostimulator 12 with a magnetically-actuated reed switch, such as described in commonly-assigned U.S. Patent applications, Serial Nos. 13/314,130 and 13/352,244, cited supra. In addition, the delivery of the enhanced dose and the maintenance dose can also be manually swapped. For instance, the switch can be used when the maintenance dose is tolerable to the patient 10, while the enhanced dose and the restorative dose are intolerable. Other uses of the switch are possible.

The recordable memory 29 in the electronic circuitry 22 of the neurostimulator 12 (shown in FIGURE 2A) stores the stimulation parameters that control the overall functionality of the pulse generator 11 in providing VNS therapy. FIGURE 13 is a flow diagram showing a routine 290 for storing operating modes for use with the method 270 of FIGURE 12. Two operating modes are stored, which include a maintenance dose of VNS tuned to restore cardiac autonomic balance (step 291) through continuously-cycling, intermittent and periodic electrical pulses, and an enhanced dose tuned to prevent initiation of or disrupt tachyarrhythmia (step 292) through periodic electrical pulses delivered at higher intensity than the maintenance dose.

In one embodiment, the autonomic regulation therapy is provided in a low level maintenance dose independent of cardiac cycle to activate both parasympathetic afferent and efferent neuronal fibers in the vagus nerve simultaneously and a high level enhanced dose. In the maintenance dose, a pulse width in the range of 250 to 500 µsec delivering between 0.02 and 1.0 mA of output current at a signal frequency in the range of 10 to 20 Hz, and a duty cycle of 5 to 30%, although other therapeutic values could be used as appropriate.

Different enhanced doses can be provided to respond to different tachyarrhythmic events. The enhanced dose settings are physician-programmable. For a default enhanced dose, the stimulation parameters would be in the same range as the maintenance dose, but would be moderately higher, with a pulse width again in the range of 250 to 500 µsec delivering between 1.5 and 2.0 mA of output current at a signal frequency in the range of 10 to 20 Hz. The duty cycle may change significantly from nominally 10% to temporarily 50% or 100%, although other therapeutic values could be used as appropriate. For non-life-threatening or non-paroxysmal tachyarrhythmias, the intensity of the enhanced dose is progressively increased over time by increasing output current, duty cycle, or frequency, lengthening pulse width, or through a combination of the foregoing parameters. Discretely-defined enhanced doses, each using different parameters sets, may be delivered in the course of treating a single continuing tachyarrhythmic event, such as for life-threatening or paroxysmal arrhythmias that rapidly generate and require a significantly strongly enhanced dose with no ramp up time.

In a further embodiment, the suspension and resumption of the enhanced dose and, in a further embodiment, the maintenance dose, can be titrated to gradually withdraw or introduce their respective forms of VNS.

## Claims

1. An implantable neurostimulator (11) comprising:
a pulse generator (12) configured to deliver electrical therapeutic stimulation in a manner that results in creation and propagation in both afferent and efferent directions of action potentials within neuronal fibers comprising a patient's cervical vagus nerve and to store operating modes of the pulse generator in a recordable i Z memory,
wherein a maintainance dose tuned to restore cardiac autonomic balance through continuously-cycling, intermittent and periodic electrical pulses to be used if no tachyarrhythmia is present is parametrically defined in the recordable memory, and
an enhanced dose tuned to prevent initiation of or disrupt tachyarrhythmia upon the patient's awakening from a sleep state through at least one of continuously-cycling, intermittent and periodic electrical pulses is parametrically defined in the recordable memory and
wherein the maintenance dose of the electrical therapeutic stimulation is at a lower intensity than the enhanced dose; and
a sensor (31, 32, 33) configured to monitor the patient's physiological state, and at least a pair of helical electrodes (14) electrically coupled to the pulse generator (12) via a nerve stimulation therapy lead(13); and wherein the pulse generator (12) is configured to deliver for a period of time the enhanced dose to the vagus nerve through the at least one pair of helical electrodes (14) upon sensing the patient's awakening from a sleep state.

2. An implantable neurostimulator according to Claim 1,
wherein the at least one sensor is a heart rate sensor (31) that is configured to
establish a normative heart rate of the patient as a mean heart rate sensed during the sleep state; and
periodically sense the patient's heart rate; and
confirm that the patient is awakening from the sleep state when the patient's heart rate gradually rises and is sustained at an elevated heart rate above the normative heart rate by a threshold amount.

3. An implantable neurostimulator according to Claim 1, wherein the at least one sensor is an accelerometer (32) configured to
establish a normative activity level of the patient as a mean frequency of movement sensed during the sleep state; and
periodically sense the patient's activity level with the accelerometer and
confirm that the patient is awakening from the sleep state when the when the patient's activity level gradually rises and is sustained at an elevated activity level above the normative activity level accompanied by an increased frequency of movement by a threshold amount.

4. An implantable neurostimulator according to Claim 1, wherein the at least one sensor is a minute ventilation sensor (33) that is configured to
establish a normative tidal volume and normative respiratory rate of the patient with the minute ventilation sensor sensed during the sleep state; and
periodically sense the patient's tidal volume and respiratory rate; and
confirm that the patient is awakening from the sleep state when the when the patient's tidal volume and respiratory rate gradually rise and are sustained at elevated levels respectively above the normative tidal volume and the normative respiratory rate by a threshold amount.

5. An implantable neurostimulator according to Claim 1, further configured to
monitor the patient's normative physiology during the delivery of the enhanced dose, and upon sensing a condition indicative of an onset of tachyarrhythmia, intensify the electrical therapeutic stimulation as specified in the operating mode.

6. An implantable neurostimulatoraccording to Claim 5, further configured to progressively intensify the electrical therapeutic stimulation as specified in the operating mode as the tachyarrhythmia continues.

7. An implantable neurostimulator according to Claim 5, further configured to maximize the electrical therapeutic stimulation as specified in the operating mode when the tachyarrhythmia fails to respond to the intensified electrical therapeutic stimulation.

8. An implantable neurostimulator according to Claim 1, further configured to
extend the period of time of enhanced dose delivery based on the patient's heart response trajectory.

9. An implantable neurostimulator according to Claim 1, further configured to
progressively intensify the electrical therapeutic stimulation as specified in the operating mode independently of the patient's heart response trajectory.

10. An implantable neurostimulator according to Claim 1, wherein the pulse generator is configured to
store the operating modes in the recordable memory by:
parametrically defining a restorative dose of the electrical therapeutic stimulation tuned to prevent initiation of or disrupt tachyarrhythmia through periodic electrical pulses delivered at a higher intensity than the maintenance dose; and wherein the pulse generator is configured to
electrically stimulate the cervical vagus nerve after an expiration of the period of time, comprising one of:
upon detecting the presence of the condition indicative of the tachyarrhythmia after an expiration of the period of time, switching to delivering the restorative dose to the vagus nerve through the pair of helical electrodes; and
upon detecting an absence of the condition indicative of tachyarrhythmia after the expiration of the time period, switch to delivering the maintenance dose to the vagus nerve through the pair of helical electrodes.

11. An implantable neurostimulator according to Claim 10, wherein
the intensity of the enhanced dose is defined as comprising one or more of an output current, a duty cycle, a frequency, and a pulse width;
the lower intensity of the maintenance dose is defined as comprising one or more of an output current lower than the enhanced dose output current, a duty cycle lower than the enhanced dose duty cycle, a frequency lower than the enhanced dose frequency, and a pulse width shorter than the enhanced dose pulse width; and
the higher intensity of the restorative dose is defined as comprising one or more of an output current higher than the maintenance dose output current, a duty cycle higher than the maintenance dose duty cycle, a frequency higher than the maintenance dose frequency, and a pulse length longer than the maintenance pulse length.

12. An implantable neurostimulator according to Claim 10, further comprising
a magnetically-actuatable reed switch configured to control the pulse generator in response to a magnetic signal remotely applied to the reed switch by at least one of:
switching between delivery of the maintenance dose, the enhanced dose, and the restorative dose;
triggering or increasing delivery of either the maintenance dose, the enhanced dose, and the restorative dose; and
decreasing or suspending delivery of either the maintenance dose, the enhanced dose, and the restorative dose.

13. An implantable neurostimulator according to Claim 1, further configured to
titrate the electrical therapeutic stimulation when beginning or suspending the delivery of the enhanced dose.

14. An implantable neurostimulator according to Claim 1, wherein the pulse generator is configured to define the enhanced dose by at least one of:
monitoring the patient's normative physiology during the delivery of the enhanced dose, and suspending the electrical therapeutic stimulation as specified in the operating mode upon a presence of a bradyarrhythmia.

15. A non-transitory computer readable storage medium storing code for executing on an implantable neurostimulator comprising a pulse generator configured to deliver electrical therapeutic stimulation in a manner that results in creation and propagation in both afferent and efferent directions of action potentials within neuronal fibers comprising a patient's cervical vagus nerve to perform, when run on the implantable neurostimulator, the steps of:
storing operating modes of the pulse generator in a recordable memory,
wherein a maintainance dose tuned to restore cardiac autonomic balance through continuously-cycling, intermittent and periodic electrical pulses to be used if no tachyarrhythmia is present is parametrically defined in the recordable memory, and
an enhanced dose tuned to prevent initiation of or disrupt tachyarrhythmia upon the patient's awakening from a sleep state through at least one of continuously-cycling, intermittent and periodic electrical pulses is parametrically defined in the recordable memory and
wherein the maintenance dose of the electrical therapeutic stimulation is at a lower intensity than the enhanced dose; and
monitoring the patient's physiological state via at least one sensor comprised in the implantable neurostimulator, and upon sensing the patient's awakening from a sleep state, delivering for a period of time the enhanced dose to the vagus nerve via the pulse generator through at least a pair of helical electrodes electrically coupled to the pulse generator via a nerve stimulation therapy lead.

## Patentansprüche

1. Ein implantierbarer Neurostimulator (11), der umfasst:
einen Pulsgenerator (12), der dazu ausgebildet ist, eine elektrische therapeutische Stimulation in einer Weise zu verabreichen, die in einer Erzeugung und Propagation sowohl in eine afferente als auch eine efferente Richtung von Aktionspotentialen in neuronalen Fasern, die einen zervikalen Vagusnerv eines Patienten umfassen, resultiert, und Betriebsmodi des Pulsgenerators in einem beschreibbaren Speicher zu speichern, wobei in dem beschreibbaren Speicher eine Erhaltungsdosis, die darauf abgestimmt ist, eine kardiale autonome Balance durch kontinuierlich-zyklische, intermittierende und periodische elektrische Pulse wiederherzustellen, die zu verwenden sind, wenn keine Tachiarrhythmie vorliegt, parametrisch definiert ist, und
eine erhöhte Dosis, die darauf abgestimmt ist, durch zumindest eines von kontinuierlich-zyklischen, intermittierenden und periodischen elektrischen Pulsen das Einsetzen von Tachiarrhythmie zu verhindern oder Tachiarrhythmie zu unterbrechen, wenn der Patient aus einem Schlafzustand aufwacht, in dem beschreibbaren Speicher parametrisch definiert ist, und
wobei die Erhaltungsdosis der elektrischen therapeutischen Stimulation von geringerer Intensität als die erhöhte Dosis ist, und
einen Sensor (31, 32, 33), der dazu ausgebildet ist, den physiologischen Zustand des Patienten zu überwachen, und zumindest ein Paar helischer Elektroden (14), die über ein Nervenstimulationstherapiekabel (13) mit dem Pulsgenerator (12) elektrisch verbunden sind; und
wobei der Pulsgenerator (12) dazu ausgebildet ist, für eine Zeitdauer die erhöhte Dosis über das zumindest eine Paar helischer Elektroden (14) an den Vagusnerv zu liefern, wenn detektiert wird, dass der Patient aus einem Schlafzustand erwacht.

2. Ein implantierbarer Neurostimulator gemäß Anspruch 1, in dem der zumindest eine Sensor ein Herzratensensor (31) ist, der dazu ausgebildet ist, ein normative Herzrate des Patienten als eine mittlere Herzrate, die während des Schlafzustands detektiert wird, festzuschreiben; und
periodisch die Herzrate des Patienten zu detektieren; und
zu bestätigen, dass der Patient aus dem Schlafzustand aufwacht, wenn die Herzrate des Patienten sukzessive ansteigt und um einen vorbestimmten Schwellenbetrag auf einer erhöhten Herzrate oberhalb der normativen Herzrate verbleibt.

3. Ein implantierbarer Neurostimulator gemäß Anspruch 1, in dem der zumindest eine Sensor ein Beschleunigungsmesser (32) ist, der dazu ausgebildet ist, ein normatives Aktivitätsniveau des Patienten als eine durchschnittliche Frequenz von Bewegungen, die während des Schlafzustands detektiert werden, festzuschreiben; und
mithilfe des Beschleunigungsmessers periodisch das Aktivitätsniveau des Patienten zu detektieren und
zu bestätigen, dass der Patient aus dem Schlafzustand aufwacht, wenn das Aktivitätsniveau des Patienten sukzessive ansteigt und um einen vorbestimmten Schwellenbetrag auf einem erhöhten Aktivitätsniveau oberhalb des normativen Aktivitätsniveaus begleitet von einer erhöhten Frequenz von Bewegungen verbleibt.

4. Ein implantierbarer Neurostimulator gemäß Anspruch 1, in dem der zumindest eine Sensor ein Atemminutenvolumensensor (33) ist, der dazu ausgebildet ist, ein normatives Atemzugvolumen und eine normative Atemfrequenz des Patienten, die während des Schlafzustands mit dem Atemminutenvolumensensor detektiert werden, festzuschreiben; und
periodisch das Atemzugvolumen und die normative Atemfrequenz des Patienten zu detektieren; und
zu bestätigen, dass der Patient aus dem Schlafzustand aufwacht, wenn das Atemzugvolumen und die Atemfrequenz Aktivitätsniveau sukzessive ansteigen und um einen vorbestimmten Schwellenbetrag jeweils auf erhöhten Niveaus oberhalb des normativen Atemzugvolumens und der normativen Atemfrequenz verbleiben.

5. Ein implantierbarer Neurostimulator gemäß Anspruch 1, der weiterhin dazu ausgebildet ist, die normative Physiologie des Patienten während der Verabreichung der erhöhten Dosis zu überwachen und bei Detektieren einer Bedingung, die den Einsatz von Tachiarrhythmie anzeigt, die elektrische therapeutische Stimulation, wie sie in dem Betriebsmodus spezifiziert ist, zu intensivieren.

6. Ein implantierbarer Neurostimulator gemäß Anspruch 5, der weiterhin dazu ausgebildet ist, die elektrische therapeutische Stimulation, wie sie in dem Betriebsmodus spezifiziert ist, zu fortschreitend intensivieren, wenn sich die Tachiarrhythmie fortsetzt.

7. Ein implantierbarer Neurostimulator gemäß Anspruch 5, der weiterhin dazu ausgebildet ist, die elektrische therapeutische Stimulation, wie sie in dem Betriebsmodus spezifiziert ist, zu maximieren, wenn die Tachiarrhythmie nicht auf die intensivierte elektrische therapeutische Stimulation reagiert.

8. Ein implantierbarer Neurostimulator gemäß Anspruch 1, der weiterhin dazu ausgebildet ist, die Zeitdauer der Abgabe der erhöhten Dosis auf der Grundlage der Herzreaktionsentwicklung auszuweiten.

9. Ein implantierbarer Neurostimulator gemäß Anspruch 1, der weiterhin dazu ausgebildet ist, die elektrische therapeutische Stimulation, wie sie in dem Betriebsmodus spezifiziert ist, unabhängig von der Herzreaktionsentwicklung fortschreitend zu intensivieren.

10. Ein implantierbarer Neurostimulator gemäß Anspruch 1, in dem der Pulsgenerator dazu ausgebildet ist, die Betriebsmodi in dem beschreibbaren Speicher durch:
parametrisches Definieren einer Wiederherstellungsdosis der elektrischen therapeutischen Stimulation, die darauf abgestimmt ist, durch periodische elektrische Pulse, die mit einer höheren Intensität als die Erhaltungsdosis abgegeben werden, das Einsetzen von Tachiarrhythmie zu verhindern oder Tachiarrhythmie zu unterbrechen, zu speichern; und wobei der Pulsgenerator dazu ausgebildet ist,
den zervikalen Vagusnerv nach Ablauf der Zeitdauer elektrisch zu stimulieren mit
Schalten darauf, über das Paar helischer Elektroden die Wiederherstellungsdosis an den Vagusnerv zu liefern, wenn das Vorliegen der Bedingung, die die Tachiarrhythmie anzeigt, nach Ablauf der Zeitdauer detektiert wird; oder
Schalten darauf, über das Paar helischer Elektroden die Erhaltungsdosis an den Vagusnerv zu liefern, wenn das Nichtvorliegen der Bedingung, die die Tachiarrhythmie anzeigt, nach Ablauf der Zeitdauer detektiert wird.

11. Ein implantierbarer Neurostimulator gemäß Anspruch 10, wobei
die Intensität der erhöhten Dosis dadurch definiert ist, dass sie eines oder mehreres von einem Ausgangsstrom, einem Arbeitszyklus, einer Frequenz und einer Pulsbreite umfasst;
die niedrigere Intensität der Erhaltungsdosis dadurch definiert ist, dass sie eines oder mehreres von einem Ausgangsstrom, der niedriger als der Ausgangsstrom der erhöhten Dosis ist, einem Arbeitszyklus, der kürzer als der Arbeitszyklus der erhöhten Dosis ist, einer Frequenz, die niedriger als die Frequenz der erhöhten Dosis ist, und einer Pulsbreite, die kleiner als der Pulsbreite der erhöhten Dosis ist, umfasst; und
die höhere Intensität der Wiederherstellungsdosis dadurch definiert ist, dass sie eines oder mehreres von einem Ausgangsstrom, der höher als der Ausgangsstrom der Erhaltungsdosis ist, einem Arbeitszyklus, der länger als der Arbeitszyklus der Erhaltungsdosis ist, einer Frequenz, die höher als die Frequenz der Erhaltungsdosis ist, und einer Pulslänge, die größer als der Pulslänge der Erhaltungsdosis ist, umfasst.

12. Ein implantierbarer Neurostimulator gemäß Anspruch 10, der weiterhin umfasst
einen magnetisch schaltbaren Reed-Schalter, der dazu ausgebildet ist, den Pulsgenerator in Reaktion auf ein magnetisches Signal, das von Ferne dem Reed-Schalter zugeführt wird, zumindest durch eines von dem Folgenden zu steuern:
Schalten zwischen dem Liefern von der Erhaltungsdosis, der erhöhten Dosis und der Wiederherstellungsdosis;
Triggern oder Verstärken des Lieferns von einer der Erhaltungsdosis, der erhöhten Dosis und der Wiederherstellungsdosis; und
Abschwächen oder Aussetzen des Lieferns von einer der Erhaltungsdosis, der erhöhten Dosis und der Wiederherstellungsdosis.

13. Ein implantierbarer Neurostimulator gemäß Anspruch 1, der weiterhin dazu ausgebildet ist,
die elektrische therapeutische Stimulation beim Starten oder Aussetzen des Lieferns der erhöhten Dosis zu dosieren.

14. Ein implantierbarer Neurostimulator gemäß Anspruch 1, wobei der Pulsgenerator dazu ausgebildet ist, die erhöhte Dosis durch zumindest eines von dem Folgenden zu bestimmen:
Überwachen der normativen Physiologie des Patienten während des Lieferns der erhöhten Dosis und Aussetzens der elektrischen therapeutischen Stimulation, wie sie in dem Betriebsmodus spezifiziert ist, bei Auftreten von Bradyarrhythmia.

15. Ein nicht-flüchtiges computerlesbares Speichermedium, das einen Code zum Ausführen auf einem implantierbaren Neurostimulator, der einen Pulsgenerator umfasst, der dazu ausgebildet ist, eine elektrische therapeutische Stimulation in einer Weise zu verabreichen, die in einer Erzeugung und Propagation sowohl in eine afferente als auch eine efferente Richtung von Aktionspotentialen in neuronalen Fasern, die einen zervikalen Vagusnerv eines Patienten umfassen, resultiert, speichert, um, wenn er auf dem implantierbaren Neurostimulator laufengelassen wird, die Schritte auszuführen:
Speichern von Betriebsmodi des Pulsgenerators in einen beschreibbaren Speicher, wobei eine Erhaltungsdosis, die darauf abgestimmt ist, eine kardiale autonome Balance durch kontinuierlich-zyklische, intermittierende und periodische elektrische Pulse wiederherzustellen, die zu verwenden sind, wenn keine Tachiarrhythmie vorliegt, in dem Speicher parametrisch definiert ist, und
eine erhöhte Dosis, die darauf abgestimmt ist, durch zumindest eines von kontinuierlich-zyklischen, intermittierenden und periodischen elektrischen Pulsen das Einsetzen von Tachiarrhythmie zu verhindern oder Tachiarrhythmie zu unterbrechen, wenn der Patient aus einem Schlafzustand aufwacht, in dem beschreibbaren Speicher parametrisch definiert ist, und
wobei die Erhaltungsdosis der elektrischen therapeutischen Stimulation von geringerer Intensität als die erhöhte Dosis ist, und
Überwachen des physiologischen Zustands des Patienten mit zumindest einem in dem implantierbaren Neurostimulator enthaltenen Sensor, und bei Detektieren davon, dass der Patient aus einem Schlafzustand aufwacht, Verabreichen der erhöhten Dosis über eine Zeitdauer an den Vagusnerv mit dem Pulsgenerator über zumindest ein Paar helischer Elektroden, die über ein Nervenstimulationstherapiekabel mit dem Pulsgenerator elektrisch verbunden sind.

## Revendications

1. Neurostimulateur implantable (11) comprenant :
un générateur d'impulsion (12) configuré pour administrer la stimulation thérapeutique électrique d'une manière qui résulte en la création et la propagation à la fois dans les sens afférents et efférents des potentiels d'action à l'intérieur des fibres neuronales comprenant un nerf vague cervical du · de la patient ·e et pour stocker les modes de fonctionnement du générateur d'impulsion dans une mémoire enregistrable,
une dose d'entretien ajustée pour restaurer l'équilibre autonome cardiaque à travers des impulsions électriques intermittentes et périodiques, à cyclage continu à utiliser si aucune tachyarythmie n'est présente est paramétriquement définie dans la mémoire enregistrable, et
une dose renforcée ajustée pour prévenir l'initiation de, ou rompre, la tachyarythmie lors de l'éveil du ·de la patient ·e d'un état de sommeil à travers au moins l'une des impulsions électriques intermittentes et périodiques, à cyclage continu est paramétriquement définie dans la mémoire enregistrable et
la dose d'entretien de la stimulation thérapeutique électrique se trouve à une intensité moindre par rapport à la dose renforcée ; et
un capteur (31, 32, 33) configuré pour surveiller l'état physiologique du ·de la patient ·e, et au moins une paire d'électrodes hélicoïdales (14) électriquement accouplées au générateur d'impulsion (12) par l'intermédiaire d'un fil de thérapie de stimulation de nerf (13) ; et
le générateur d'impulsion (12) étant configuré pour l'administration sur une période de temps de la dose renforcée au nerf vague à travers la au moins une paire d'électrodes hélicoïdales (14) lors de la détection de l'éveil du ·de la patient ·e d'un état de sommeil.

2. Neurostimulateur implantable selon la revendication 1,
le au moins un capteur étant un capteur de fréquence cardiaque (31) qui est configuré pour établir une fréquence cardiaque normative du ·de la patient ·e comme fréquence cardiaque moyenne détectée durant l'état de sommeil ; et
détecter périodiquement la fréquence cardiaque du ·de la patient ·e ; et
confirmer que le · la patient ·e s'éveille de l'état de sommeil lorsque la fréquence cardiaque du ·de la patient ·e s'élève graduellement et est maintenue à une fréquence cardiaque élevée au-dessus de la fréquence cardiaque normative d'une quantité seuil.

3. Neurostimulateur implantable selon la revendication 1, le au moins un capteur étant un accéléromètre (32) configuré pour établir un niveau d'activité normatif du ·de la patient ·e comme fréquence moyenne de mouvement détecté durant l'état de sommeil ; et
détecter périodiquement le niveau d'activité du ·de la patient ·e avec l'accéléromètre et
confirmer que le · la patient ·e s'éveille de l'état de sommeil lorsque lorsque le niveau d'activité du ·de la patient ·e s'élève graduellement et est maintenu à un niveau d'activité élevé au-dessus du niveau d'activité normatif accompagné d'une fréquence accrue de mouvement d'une quantité seuil.

4. Neurostimulateur implantable selon la revendication 1, le au moins un capteur étant un capteur de ventilation instantané (33) qui est configuré pour établir un volume courant normatif et une fréquence respiratoire normative du ·de la patient ·e avec le capteur de ventilation instantané détectée durant l'état de sommeil ; et
détecter périodiquement le volume courant et la fréquence respiratoire du ·de la patient ·e ; et
confirmer que le · la patient ·e s'éveille de l'état de sommeil lorsque lorsque le volume courant et la fréquence respiratoire du ·de la patient ·e s'élève graduellement et sont maintenus à des niveaux élevés respectivement au-dessus du volume courant normatif et de la fréquence respiratoire normative d'une quantité seuil.

5. Neurostimulateur implantable selon la revendication 1, configuré en outre pour surveiller la physiologie normative du ·de la patient ·e durant l'administration de la dose renforcée, et lors de la détection d'un état indicateur d'une apparition de tachyarythmie, intensifier la stimulation thérapeutique électrique telle que spécifiée dans le mode de fonctionnement.

6. Neurostimulateur implantable selon la revendication 5, configuré en outre pour intensifier progressivement la stimulation thérapeutique électrique telle que spécifiée dans le mode de fonctionnement lorsque la tachyarythmie se poursuit.

7. Neurostimulateur implantable selon la revendication 5, configuré en outre pour maximiser la stimulation thérapeutique électrique telle que spécifiée dans le mode de fonctionnement lorsque la tachyarythmie échoue à répondre à la stimulation thérapeutique électrique intensifiée.

8. Neurostimulateur implantable selon la revendication 1, configuré en outre pour allonger la période de temps d'administration de dose renforcée sur la base de la trajectoire de réponse du cœur du ·de la patient ·e.

9. Neurostimulateur implantable selon la revendication 1, configuré en outre pour intensifier progressivement la stimulation thérapeutique électrique telle que spécifiée dans le mode de fonctionnement indépendamment de la trajectoire de réponse du cœur du ·de la patient ·e.

10. Neurostimulateur implantable selon la revendication 1, le générateur d'impulsion étant configuré pour stocker les modes de fonctionnement dans la mémoire enregistrable :
en définissant paramétriquement une dose de restauration de la stimulation thérapeutique électrique ajustée pour prévenir l'initiation de, ou rompre, la tachyarythmie à travers des impulsions électriques périodiques administrées à une intensité supérieure par rapport à la dose d'entretien ; et le générateur d'impulsion étant configuré pour stimuler électriquement le nerf vague cervical après une expiration de la période de temps, comprenant l'une parmi :
lors de la détection de la présence de l'état indicateur de la tachyarythmie après une expiration de la période de temps, le basculement vers l'administration de la dose de restauration au nerf vague à travers la paire d'électrodes hélicoïdales ; et
lors de la détection d'une absence d'état indicateur de tachyarythmie après l'expiration de la période de temps, le basculement vers l'administration de la dose d'entretien au nerf vague à travers la paire d'électrodes hélicoïdales.

11. Neurostimulateur implantable selon la revendication 10,
l'intensité de la dose renforcée étant définie comme comprenant l'un ou plusieurs parmi un courant de sortie, un cycle de charge, une fréquence, et une largeur d'impulsion ;
l'intensité moindre de la dose d'entretien étant définie comme comprenant l'un ou plusieurs d'un courant de sortie inférieur au courant de sortie de dose renforcée, d'un cycle de charge inférieur au cycle de charge de dose renforcée, d'une fréquence inférieure à la fréquence de dose renforcée, et d'une largeur d'impulsion plus courte que la largeur d'impulsion de dose renforcée ; et
l'intensité supérieure de la dose de restauration étant définie comme comprenant l'un ou plusieurs d'un courant de sortie supérieur au courant de sortie de dose d'entretien, d'un cycle de charge supérieur au cycle de charge de dose d'entretien, d'une fréquence supérieure à la fréquence de dose d'entretien, et d'une longueur d'impulsion plus longue que la longueur d'impulsion d'entretien.

12. Neurostimulateur implantable selon la revendication 10, comprenant en outre un commutateur à lames souples pouvant être magnétiquement actionné configuré pour commander le générateur d'impulsion en réponse à un signal magnétique appliqué à distance au commutateur à lames souples d'au moins l'un parmi :
la commutation entre l'administration de la dose d'entretien, de la dose renforcée, et de la dose de restauration ;
le déclenchement ou l'accroissement de l'administration de l'une ou l'autre de la dose d'entretien, de la dose renforcée, et de la dose de restauration ; et
la baisse ou la suspension de l'administration de l'une ou l'autre de la dose d'entretien, de la dose renforcée, et de la dose de restauration.

13. Neurostimulateur implantable selon la revendication 1, configuré en outre pour titrer la stimulation thérapeutique électrique lors du début ou de la suspension de l'administration de la dose renforcée.

14. Neurostimulateur implantable selon la revendication 1, le générateur d'impulsion étant configuré pour définir la dose renforcée d'au moins l'une parmi :
la surveillance de la physiologie normative du ·de la patient ·e durant l'administration de la dose renforcée, et
la suspension de la stimulation thérapeutique électrique telle que spécifiée dans le mode de fonctionnement lors de la présence d'une bradyarythmie.

15. Code de stockage de milieu de stockage non temporaire lisible par ordinateur pour l'exécution sur un neurostimulateur implantable comprenant un générateur d'impulsion configuré pour administrer la stimulation thérapeutique électrique d'une manière qui résulte en création et propagation dans à la fois les sens afférents et efférents des potentiels d'action à l'intérieur des fibres neuronales comprenant un nerf vague cervical du ·de la patient ·e pour effectuer, lors de l'exécution sur le neurostimulateur implantable, les étapes de :
stockage des modes de fonctionnement du générateur d'impulsion dans une mémoire enregistrable, une dose d'entretien ajustée pour restaurer l'équilibre autonome cardiaque à travers des impulsions électriques intermittentes et périodiques, à cyclage continu à utiliser si aucune tachyarythmie n'est présente est paramétriquement définie dans la mémoire enregistrable, et
une dose renforcée ajustée pour prévenir l'initiation de, ou rompre, la tachyarythmie lors de l'éveil du ·de la patient ·e d'un état de sommeil à travers au moins l'une des impulsions électriques intermittentes et périodiques, à cyclage continu est paramétriquement définie dans la mémoire enregistrable et
la dose d'entretien de la stimulation thérapeutique électrique se trouve à une intensité moindre par rapport à la dose renforcée ; et
de surveillance de l'état physiologique du ·de la patient ·e par l'intermédiaire d'au moins un capteur compris dans le neurostimulateur implantable, et lors de la détection de l'éveil du ·de la patient ·e d'un état de sommeil, l'administration sur une période de temps de la dose renforcée au nerf vague par l'intermédiaire du générateur d'impulsion à travers au moins une paire d'électrodes hélicoïdales électriquement accouplée au générateur d'impulsion par l'intermédiaire d'un fil de thérapie de stimulation de nerf.
